Europäisches Patentamt

European Patent Office

Office européen des brevets

(11) Numéro de publication: **0 235 004 B1**

# FASCICULE DE BREVET EUROPEEN

(45) Date de publication de fascicule du brevet: **30.06.93** (51) Int. Cl.5: **C12Q 1/70**, C12N 15/00

(21) Numéro de dépôt: **87400218.1**

(22) Date de dépôt: **30.01.87**

(54) **Sondes à papillomavirus et procédé de diagnostic in vitro d'infections à papillomavirus.**

(30) Priorité: **31.01.86 FR 8601425**

(43) Date de publication de la demande:
**02.09.87 Bulletin 87/36**

(45) Mention de la délivrance du brevet:
**30.06.93 Bulletin 93/26**

(84) Etats contractants désignés:
**AT BE CH DE ES FR GB GR IT LI LU NL SE**

(56) Documents cités:
**EP-A- 0 192 001**

**JOURNAL OF VIROLOGY, vol. 52, no. 3, décembre 1984, American Society for Microbiology (US); D.KREMSDORF et al., pp. 1013-1018***

**CHEMICAL ABSTRACTS, vol. 108, no. 7, février 1988, Columbus, OH (US); S.BEAUDENON et al., p. 396, no. 52535w***

(73) Titulaire: **INSTITUT PASTEUR**
**25-28, rue du Docteur Roux**
**F-75724 Paris Cédex 15(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Kremsdof, Dina**
**35, rue Esquirol**
**F-75013 Paris(FR)**
Inventeur: **Croissant, Odile**
**19, rue Auguste Lançon**
**F-75013 Paris(FR)**
Inventeur: **Orth, Gérard**
**49, rue du Dr. Roux**
**F-92200 Sceaux(FR)**
Inventeur: **Baudenon, Sylvie**
**13 bis, rue des Truilles**
**92140 Clamart(FR)**

(74) Mandataire: **Gutmann, Ernest et al**
**S.C. Ernest Gutmann - Yves Plasseraud 67, boulevard Haussmann**
**F-75008 Paris (FR)**

## Description

L'invention concerne des ADNs de papillomavirus, et plus particulièrement des sondes dérivées de ces papillomavirus, ainsi que des procédés les mettant en oeuvre pour le diagnostic in vitro d'infections à papillomavirus.

L'expression "papillomavirus" recouvre un grand nombre de virus ayant en commun d'être tenus pour responsables de plusieurs formes d'infections virales s'étageant entre les verrues cutanées ou de muqueuses, relativement bénignes, et des hyperplasies susceptibles de dégénérer en néoplasies intra-épithéliales et en cancers de la peau et des muqueuses. Parmi des infections à papillomavirus, on mentionnera également plus particulièrement l'épidermodysplasie verruciforme, qui sera quelquefois ci-après désignée sous l'expression "EV".

Un certain nombre de types de papillomavirus a déjà été décrit. La demande de brevet européen n° 85.402362.9 déposée le 29 novembre 1985 et publiée le 10 septembre 1986 sous le n° 0192001, décrit un certain nombre de types et sous-types nouveaux de papillomavirus qui ont été isolés à partir de verrues, de lésions maculaires, susceptibles de donner lieu au développement de cancers de la peau chez d'importantes proportions des malades qui en sont affectés, et à partir de lésions pré-cancéreuses de la peau, de lésions d'hyperplasies orales et d'un cancer du col de l'utérus.

Le rôle majeur de divers types de virus de papillomes humains (HPV) dans la genèse des néoplasies a été reconnu. leur pathogénicité est également influencée par des facteurs génétiques divers, notamment immunitaires et/ou extrinsèques, tels que des rayonnements actiniques dans la pathogénèse des infections aux papillomavirus.

Plusieurs types et sous-types de papillomavirus nouveaux ont été identifiés dans la demande antérieure. L'utilisation, dans des techniques de diagnostic in vitro plus affinées, des ADNs de ces papillomavirus nouveaux, pris isolément ou en association, entre eux et/ou avec des ADNs d'HPV antérieurement connus, a également été décrite dans cette demande.

D'une façon générale, il a été remarqué dans la demande européenne que les papillomavirus, bien que très différents entre eux, avaient des tailles de l'ordre de 7000-8000 paires de bases. En outre, leurs génomes peuvent présenter certains degrés d'homologie, évalués en pourcentages d'homologies entre types et sous-types de papillomavirus, dans des essais d'hybridation réalisés dans des conditions dites non stringentes ou non strictes ou encore dans des conditions d'hybridation stringente ou stricte.

Il a été dit que les papillomavirus qui présentent des pourcentages d'homologie inférieurs à 50 % dans des conditions strictes ou stringentes appartenaient à des types différents. Les virus pour lesquels on observe, dans ces conditions strictes ou stringentes, des pourcentages d'homologie supérieurs à 50 % sont considérés comme appartenant au même type. Ils peuvent former des sous-types différents au sein de ce même type.

Les essais d'hybridation dans les conditions non strictes ou non stringentes impliquent la mise en contact mutuelle d'ADNs provenant de deux isolats de virus dans les conditions suivantes décrites par HEILMAN C.A. et al. 1980, J. Virol., 36, 395-407, et CROISSANT et al, 1982, C.R. Acad. Sc. Paris, 294, 581-586 (molécules hétéroduplexes).

Les essais d'hybridation dans les conditions stricte ou stringentes impliquent la mise en contact mutuelle d'ADNs provenant de deux isolats de virus dans les conditions décrites par HEILMAN C.A. et al, 1980, et KREMSDORF, D. et al, ((1982), J. Virol. 43:436-447 et (1983), J. Virol. 48:340-351) et DAVIS R.W. et al, 1971, Methods Enzymol., 21:413-418 (molécules hétéroduplexes).

Les dénominations de certains des HPVs décrits dans le présent texte ont été modifiées dans la nomenclature internationale maintenant en vigueur. En particulier, les isolats identifiés dans l'énumération qui suit, chaque fois après "au lieu de" (et tels qu'ils sont désignés dans le présent texte) portent désormais les désignations indiquées à gauche :

HPV32 au lieu de HPV31 (BEAUDENON S. et al, 1987)

HPV34 au lieu de HPV32 (KAWASHIMA M. et al, 1986)

HPV33 au lieu de HPV-IP2 (BEAUDENON S. et al, 1986)

HPV36 au lieu de HPV-IP4 (KAWASHIMA M. et al, 1986)

HPV39 au lieu de HPV-IP5 (BEAUDENON S. et al, Virology 161:374-384, 1987)

HPV42 au lieu de HPV-IP6 (BEAUDENON S. el al, Virology 161:374-384, 1987).

Les désignations abrégées utiiisées dans le présent texte pour identifier divers papillomavirus ont été rendues conformes à la nomenclature internationale actuellement en vigueur. Toutefois, l'on indiquera aussi ci-après -dans les cas appropriés- les abréviations utilisées antérieurement, même encore à la date de priorité dont bénéficie le présent texte, les abréviations antérieures suivant alors immédiatement les abréviations corrigées en conformité avec les prescriptions de la nomenclature internationale. Ces abréviations antérieures sont entourées de signes de parenthèses.

Partant des précédentes considérations, plusieurs virus nouveaux ont fait l'objet d'une description dans la demande antérieure. De même ont été décrits des recombinants génétiques comprenant tout ou partie des génomes (dénommés ADN-HPVs) de ces virus. L'invention de la demande antérieure concernait par conséquent chacun des ADN-HPVs choisis parmi l'ensemble des ADNs qui présentent des tailles qui s'étagent entre 7000 et 8000 paires de bases et sont caractérisés par les cartes de restriction qui apparaissent dans les dessins également reproduits dans cette demande, en ce qui concerne plus particulièrement les ADN-HPVs obtenus à partir des papillomavirus et qui répondent aux désignations HPV2d, HPV10b, HPV14a, HPV14b, HPV15, HPV17a, HPV17b, HPV19, HPV20, HPV21, HPV22, HPV23, HPV24, HPV28, HPV29, HPV32 (HPV31), HPV34 (HPV32), HPV33 (HPV-IP2) et HPV26 (HPV-IP4).

L'invention de la demande antérieure concernait également des mélanges ou "cocktails" d'ADN-HPVs isolés à partir des papillomavirus nouveaux ou antérieurement connus, à la date de dépôt de la demande antérieure, ou des sondes d'hybridation les contenant et susceptibles d'être mis en oeuvre plus efficacement pour le diagnostic de diverses catégories d'infections, voire des niveaux de risques qu'accompagnent la découverte chez un patient de papillomavirus déterminés. Le nombre des sondes à papillomavirus décrites dans la demande antérieure, auxquelles s'ajoutent celles constituées à partir des ADNs génomiques de papillomavirus déjà isolés précédemment et leurs associations dans les mélanges déterminés, permet donc la réalisation de diagnostics affinés, notamment une grande discrimination des diverses catégories d'infections imputables aux divers types de papillomavirus ou susceptibles de se développer sous l'effet de ces derniers types et, au sein d'une catégorie d'infections déterminées, de mieux pronostiquer le degré de risque que ces dernières se transforment en des maladies plus redoutables. En particulier, l'invention de la demande antérieure fournissait des moyens permettant, dans le cas des infections se manifestant par des épidermodysplasies verruciformes, de mieux apprécier le degré de risque que ces dernières évoluent vers des cancers cutanés.

A ce titre, l'invention de la demande antérieure concernait plus particulièrement des compositions de diagnostic ; un groupe de compositions préférées était défini comme contenant :

1) au moins l'ADN de l'HPV2d,
2) au moins l'un des ADNs de HPV10b, 28 et 29,
3) au moins l'un des ADNs de HPV17, 24,
4) au moins l'un des ADNs de HPV14, 15, 17, 19, 20, 21, 22, 23 et 36 (IP4),
5) au moins l'un des ADNs de HPV15 et 17,
6) l'ADN de HPV24,
7) l'ADN de HPV14, 34 (32) et 36 (IP4),
8) l'ADN de HPV32 (31),
9) l'ADN de HPV34 (32),
10) au moins l'un des ADNs de HPV16, 18 et 33 (IP2), étant entendu que les ADNs des dix groupes étaient choisis de façon à être en toutes circonstances différents les uns des autres.

Dans le groupe 10) l'ADN de HPV33 (IP2) est, de préférence, associé à l'ADN de HPV16 ou de HPV18.

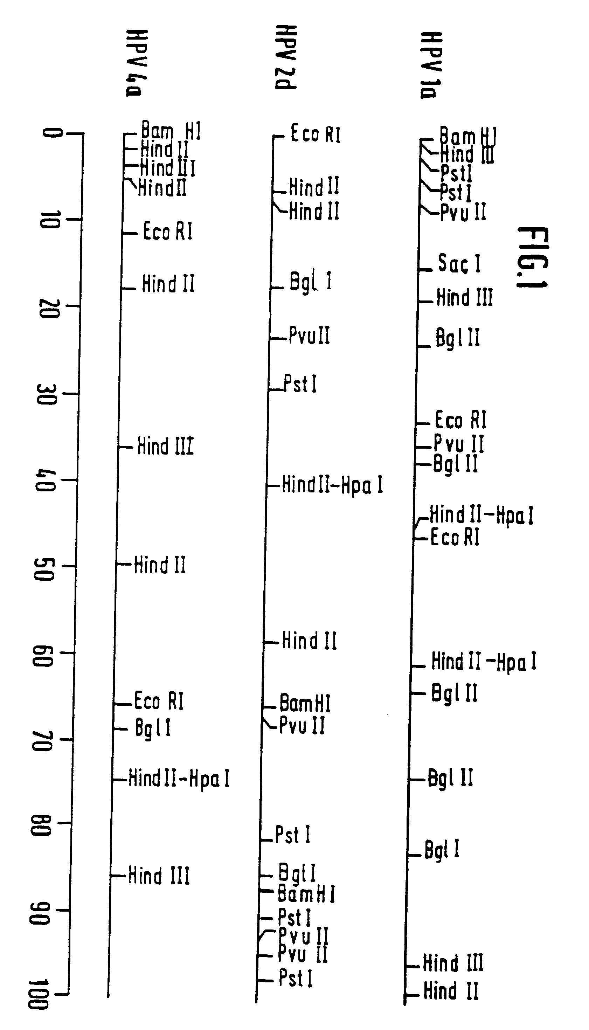

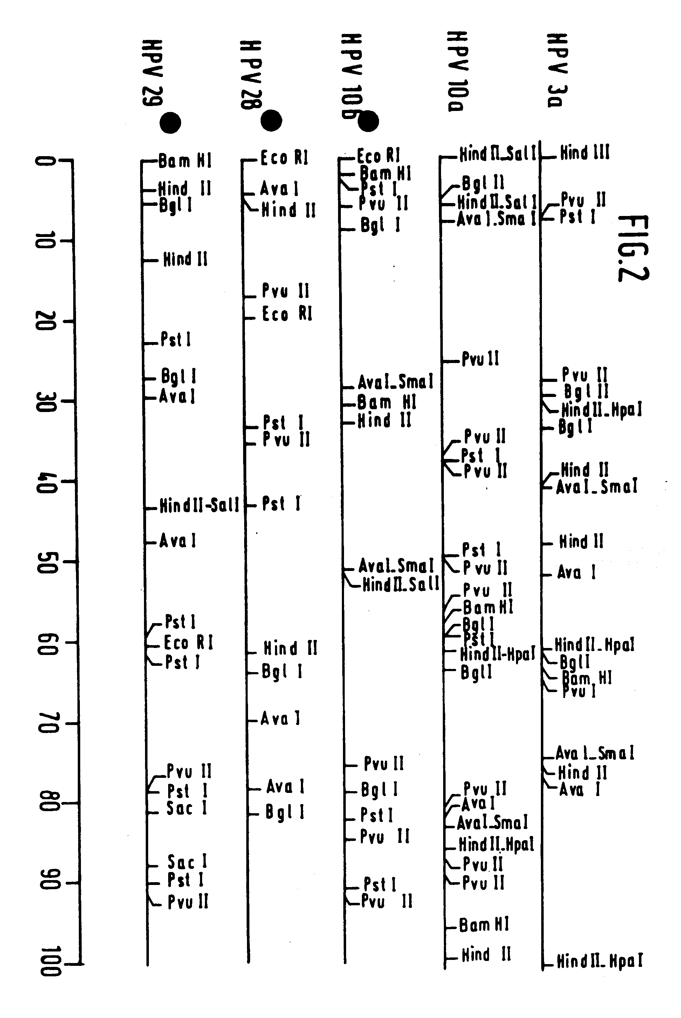

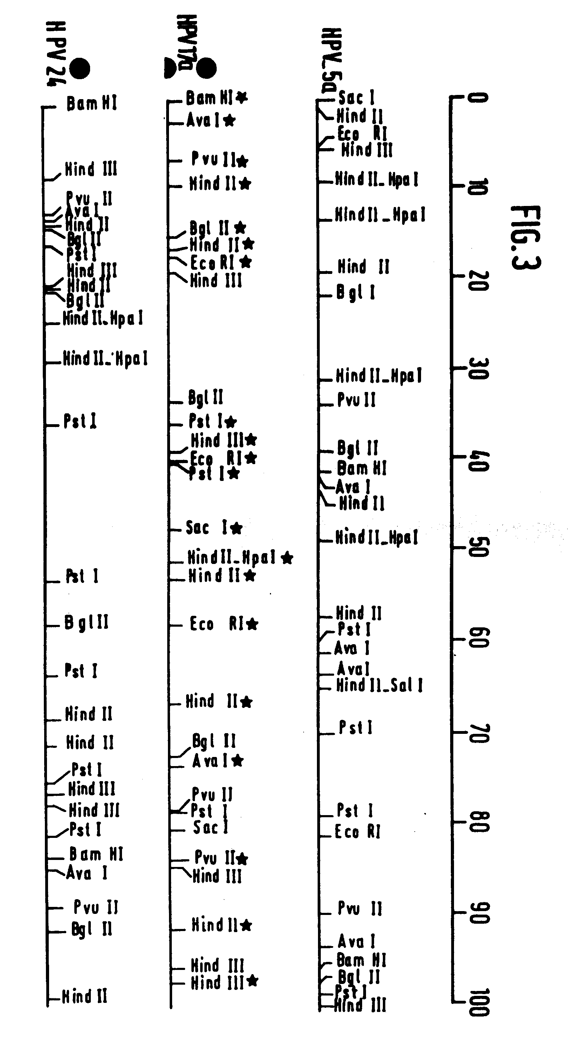

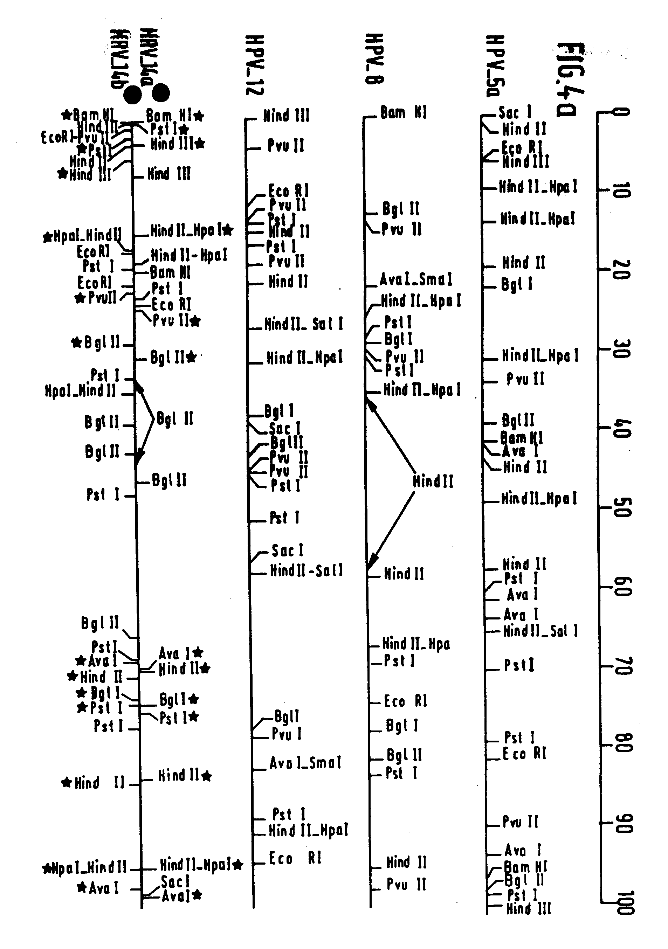

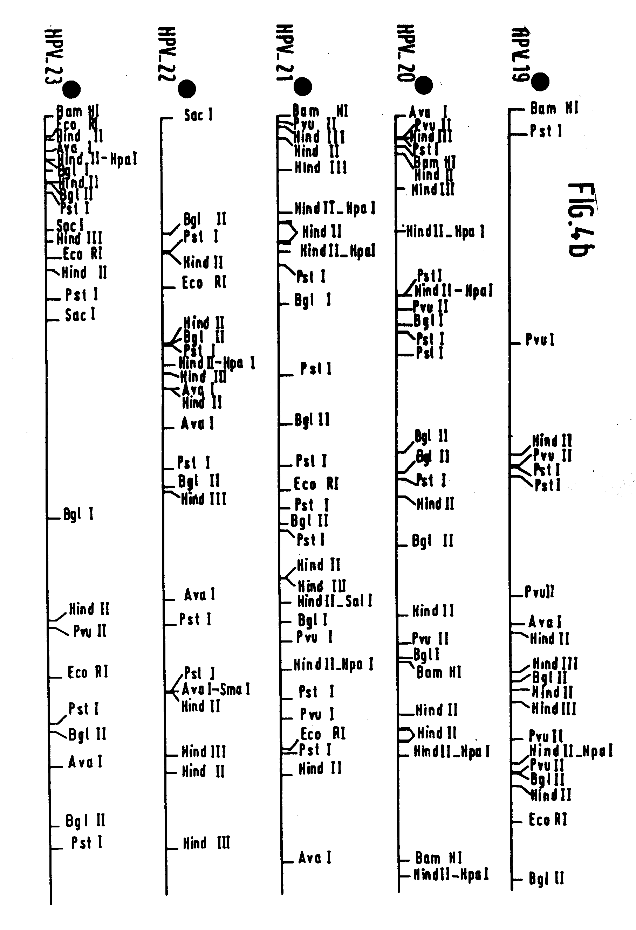

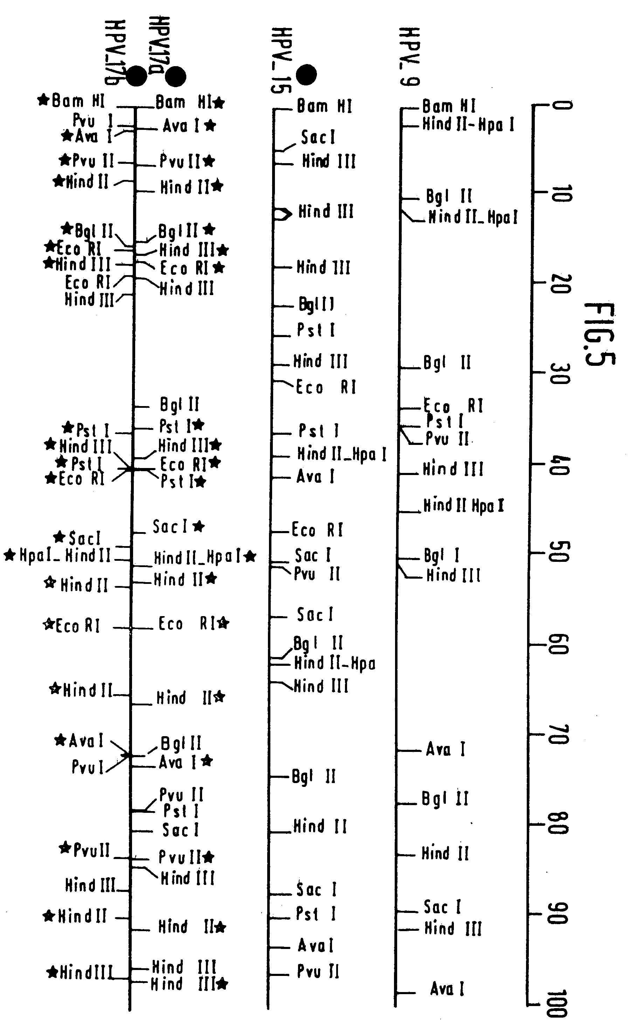

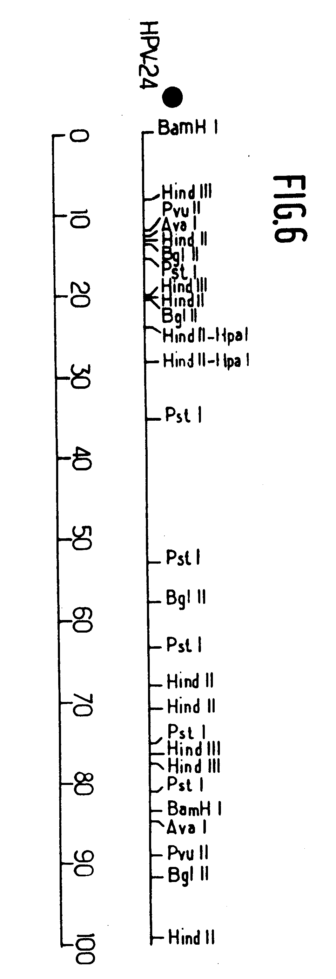

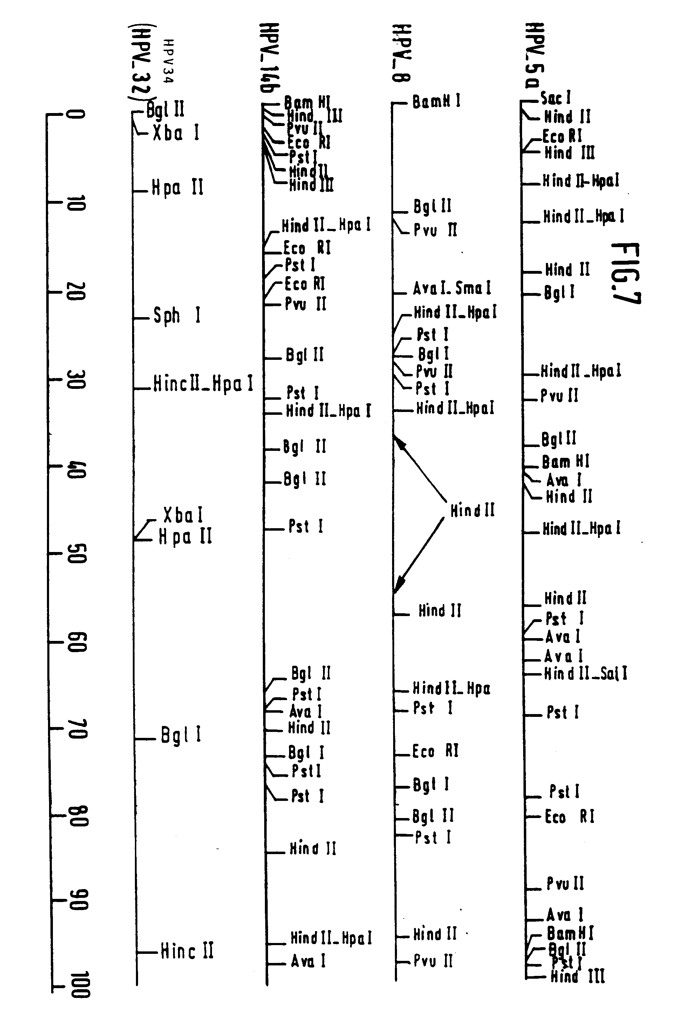

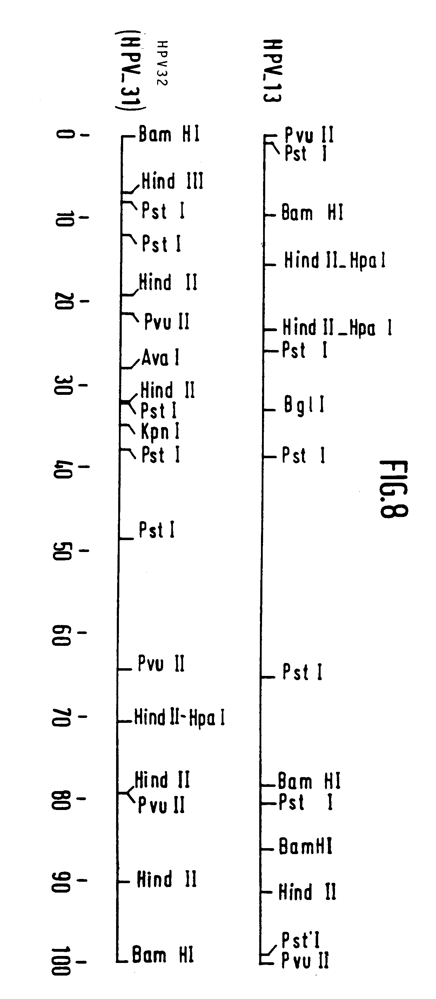

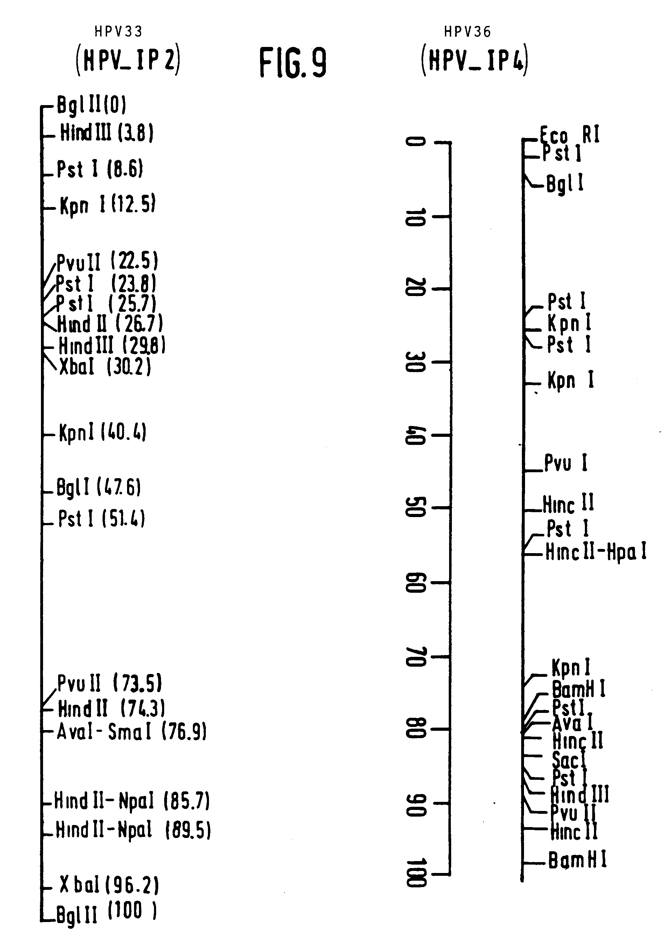

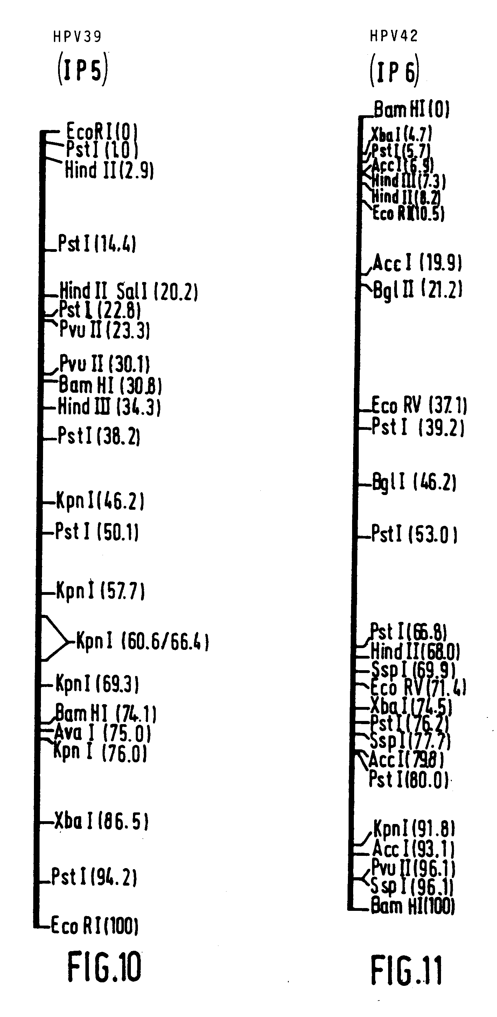

Dans les figures 1 à 10 des dessins ci-annexés, sont représentées les cartes physiques de restriction des ADN-HPVs concernés.

Les cartes physiques donnent les positions de sites de coupure par diverses endonucléases de restriction. L'origine des cartes est généralement constituée par un site unique de coupure. Les distances à l'origine sont exprimées en pourcentage de longueur de génome.

La présente invention concerne des papillomavirus nouvellement isolés, les ADNs génomiques qui peuvent en être extraits ou des fragments de ces ADNs génomiques, ainsi que de nouvelles sondes d'hybridation qui peuvent être formées à partir de ces ADN-HPVs ou fragments d'ADN-HPVs.

L'invention concerne aussi un procédé de préparation d'une sonde d'ADN-HPV, caractérisé par le clonage d'un vecteur dans un hôte approprié, ce vecteur ayant préalablement été modifié par recombinaison in vitro de tout ou partie d'un ADN d'un virus choisi parmi HPV39 (IP5) et HPV42 (IP6) et d'un vecteur approprié, pour obtenir un ADN-recombinant contenant cet ADN dans un site de ce vecteur permettant son clonage dans cet hôte, notamment dans une bactérie, et par la récupération et la purification de l'ADN recombinant cloné. Avantageusement, le vecteur utilisé est un plasmide ou un phage capable d'être répliqué dans un micro-organisme procaryote, tel que E. coli.

De préférence encore cette sonde ADN-HPV est encore marquée, pour faciliter les conditions de son utilisation. On peut avoir recours à tout principe de marquage radioactif immunofluorescent enzymatique, etc..

L'invention concerne encore des "cocktails" nouveaux de sondes les contenant ou leur utilisation dans des "cocktails" de sondes déjà décrits dans la demande antérieure, cependant complétés avec l'un ou l'autre des ADN-HPVs faisant l'objet de la présente demande et, par conséquent, des trousses ou "kits" de diagnostic encore davantage perfectionnés.

Les ADN-HPVs selon la présente invention sont caractérisés par les cartes de restrictions faisant les objets des figures 10 et 11 et respectivement dénommés HPV39 (HPV-IP5) et HPV42 (HPV IP6).

Les sondes d'hybridation construites à partir du génome de HPV39 (IP5) ou du génome de HPV42 (IP6) sont particulièrement utiles, pour le diagnostic et/ou le dépistage in vitro, dans les conditions, qui ont été décrites dans la demande antérieure et qui seront encore décrites plus loin, des types de papillomavirus qui constituent un risque pour le développement de néoplasies génitales et, en particulier, de cancers du col de l'utérus.

Ces sondes d'hybridation seront avantageusement incorporées aux mélanges de diagnostic des affections du même type sur lesquels il sera revenu plus loin.

L'invention concerne également des fragments des ADN-HPVs précédents ou capables de s'hybrider avec ceux-ci, notamment dans des conditions strictes. De même, elle concerne les ADNs recombinants contenant tout ou partie de chacun des ADN-HPVs sus-indiqués, et plus particulièrement des ADNs recombinants contenant des fragments correspondant aux gènes E1, E2, E6-E7, L1, L2 et à la région intergénique, NC, respectivement ou encore des fragments contenant des séquences correspondant aux régions intergéniques desdits ADN-HPVs. Elle concerne enfin les sondes qui peuvent être constituées à partir de ces ADN-HPVs respectifs ou à partir des fragments correspondants, et les procédés de diagnostic in vitro mettant en jeu lesdites sondes.

Les préparations d'ADN viral ont été extraites selon les techniques qui seront décrites ci-après dans les exemples 1 et 2.

Dans ce qui suit seront décrites de façon plus précise les conditions dans lesquelles les virus ont été isolés, puis les conditions dans lesquelles ont été obtenus les ADN-HPVs à partir de ces virus.

Clonage moléculaire et caractérisation d'un nouveau type d'HPV associé à des néoplasies génitales et à des cancers du col de l'utérus (HPV39) (HPV-IP5))

Un nouveau type d'HPV a été mis en évidence dans l'ADN extrait de biopsies de néoplasies intraépithéliales (papules bowenoïdes du pénis), par hybridation moléculaire sur réplique, dans des conditions non strictes, avec une sonde d'ADN radioactive spécifique du HPV6 ou du HPV10. L'étude de la sensibilité de l'ADN de cet HPV à plusieurs endonucléases de restriction a montré que l'enzyme EcoRI coupe une fois l'ADN viral. Après digestion de l'ADN extrait des lésions par l'enzyme EcoRI, la fraction renfermant des molécules d'ADN de 8 kb a été purifiée par centrifugation dans un gradient de saccharose. Les molécules d'ADN de 8 kb ont été insérées, par le site EcoRI, dans l'ADN du bactériophage λgtWESλB. Après encapsidation de l'ADN recombinant et infection de bactéries Escherichia coli K12 (souche LA101), les bactériophages recombinants ayant intégré l'ADN de HPV ont été sélectionnés par la technique d'hybridation sur plages de lyse (Benton et Davis), en utilisant une sonde d'ADN radioactive spécifique de l'ADN du HPV6, dans des conditions non strictes. Plusieurs bactériophages recombinants, contenant la totalité des séquences virales, ont été isolés. La coupure de l'ADN des bactériophages recombinants par l'enzyme d'insertion EcoRI engendre un fragment de 8 kb hybridant avec l'ADN du HPV6 dans des conditions non strictes. La coupure de l'ADN des bactériophages recombinants et de la préparation ADN extrait des lésions par le mélange des endonucléases EcoRI et PstI engendre les mêmes sept fragments, dont le poids moléculaire total correspond à celui d'un génome de HPV (8 kb).

L'ADN du nouvel HPV a été excisé des séquences de l'ADN phagique et recloné dans le plasmide pSP65. Une carte de restriction de l'ADN viral a été construite à partir de l'étude de la sensibilité de cet ADN à 16 endonucléases de restriction. 22 sites de coupure ont ainsi été localisés (fig. 10). La carte de restriction du nouvel HPV est différente de celle de tous les HPV identifiés à ce jour. Le degré d'homologie entre l'ADN du nouvel HPV et l'ADN des HPV identifiés à ce jour a été analysé par des expériences d'hybridation sur réplique, dans des conditions strictes, en utilisant une sonde radioactive préparée à partir de l'ADN purifié du nouvel HPV. Une homologie partielle a été détectée avec l'ADN du HPV18 et, à un degré moindre, du HPV26 et une homologie faible a été détectée avc l'ADN des HBVs types 2, 6, 10, 13, 29, 32 (31) et HPV33 (IP2). Le degré d'homologie entre l'ADN du nouvel HPV et l'ADN du HPV18 a été déterminé plus précisément par des expériences de réassociation ADN-ADN en milieu liquide, suivie par un traitement des hybrides par la nucléase S1. Un taux d'hybridation de 2 % a été détecté entre ces deux ADNs. Le nouveau virus caractérisé à partir de papules bowenoïdes du pénis constitue donc un nouveau type d'HPV, provisoirement dénommé HPV39 (IP5).

L'analyse, au microscope électronique, de molécules hétéroduplexes formées, dans différentes conditions, entre l'ADN de HPV39 (IP5) et l'ADN du HPV42 (16) a permis d'aligner les cartes physiques de ces deux génomes et de définir les positions théoriques de différents gènes portés par l'ADN d'HPV39 (IP5), par rapport à l'origine de la carte de cet ADN. Les positions sont exprimées en pourcentages de longueur du génome à partir de cette origine (point O sur la carte de restriction de la fig. 10).

| Localisation putative des principaux gènes et de la région intergénique (NC) du HPV39 (IP5) | |
|---|---|
| | IP5 |
| E6-E7 | 18-28 |
| E1 | 28-53 |
| E2 | 51,5-66 |
| L2 | 69,5-89 |
| L1 | 87-8 |
| NC | 7,5-18 |

L'utilisation de sondes radioatives préparées à partir de l'ADN du HPV39 (IP5) purifié a permis de déterminer le pouvoir pathogène de ce virus. Au cours d'une étude de 1522 prélèvements de cellules cervico-vaginales, destinées à préciser la fréquence de l'infection de l'épithélium du col de l'utérus par un HPV, HPV39 (IP5) a été mis en évidence dans 5 des 110 prélèvements contenant un HPV. D'autre part, HPV39 (IP5) a été détecté dans 3 des 93 biopsies de cancers invasifs du col de l'utérus analysées. Dans ces trois cas, l'intégration de la totalité des séquences d'ADN du HPV39 (IP5) dans l'ADN cellulaire a été observée.

HPV39 (IP5) constitue donc un type d'HPV génital, présentant un potentiel oncogène. Il est donc souhaitable de l'incorporer à tous mélanges d'ADN de HPV destinés à la préparation de sondes moléculaires, en vue du diagnostic ou du dépistage des types d'HPV constituant un risque pour le développement de néoplasies génitales et, en particulier, de cancers du col de l'utérus.

Clonage moléculaire et caractérisation d'un nouveau type d'HPV associé à des lésions génitales (HPV42 (IP6))

Un autre nouveau type d'HPV a été mis en évidence dans l'ADN extrait de lésions vulvaires décrites comme des papillomes montrant ds atypies cellulaires minimes, par hybridation, dans des conditions strictes, avec une sonde d'ADN radioactive spécifique du HPV32 (31). Une étude de la sensibilité de l'ADN de cet HPV à plusieurs enzymes de restriction a montré que l'enzyme BamHI coupe une fois l'ADN viral. Après digestion de l'ADN extrait des lésions par l'enzyme BamHI, les molécules d'ADN de 8 kb ont été purifiées par centrifugation dans un gradient de saccharose, puis insérées par le site BamHI dans l'ADN du bactériophage lambda L 47.1. Après encapsidation de l'ADN recombinant et infection de bactéries Escherichia coli K12 (souche LA 101), les bactériophages recombinants ayant intégré l'ADN du nouvel HPV ont été sélectionnés par la technique d'hybridation sur plages de lyse (Benton et Davis), en utilisant une sonde d'ADN radioactive spécifique du HPV32 (31) dans des conditions strictes. Plusieurs bactériophages recombinants contenant la totalité des séquences virales ont été isolés. La coupure de l'ADN des bactériophages recombinants par l'endonucléase BamHI engendre un fragment 8 kb hybridant avec l'ADN du HPV32 (31) dans des conditions strictes. La coupure de l'ADN des bactériophages recombinants et de la préparation d'ADN extrait des lésions, par le mélange des endonucléases BamHI et PstI, engendre les mêmes 7 fragments, dont le poids moléculaire total correspond à celui d'un génome de papillomavirus.

L'ADN du nouvel HPV a été excisé des séquences d'ADN phagique et recloné dans le plasmide pSP64. Une carte de restriction de l'ADN du nouvel HPV a été construite à partir de l'étude de la sensibilité de cet ADN à 20 endonucléases de restriction. 26 sites de coupure ont ainsi été localisés (fig. 10). Cette carte est différente de celle de tous les HPV isolés à ce jour. Le degré d'homologie entre l'ADN du nouvel HPV et l'ADN des HPV identifiés à ce jour a été analysé par des expériences d'hybridation sur réplique, dans des conditions strictes, en utilisant une sonde d'ADN radioactive spécifique du nouvel HPV. Une hybridation partielle a été observée avec l'ADN du HPV32 (31) et une hybridation croisée faible a été obtenue avec les ADNs des HPV6, 13 et HPV33 (IP2). Les homologies entre l'ADN du nouvel HPV et l'ADN du HPV32 (31) ont été déterminées plus précisément par réassociation ADN-ADN en milieu liquide, suivie par un traitement des hybrides par la nucléase S1. Une hybridation de 20 % a été détectée entre les deux ADNs. Le virus isolé à partir de papillomes bowenoïdes vulvaires constitue donc un nouveau type d'HPV, provisoirement dénommé HPV-IP6.

L'analyse, au microscope électronique, de molécules hétéroduplexes formées dans différentes conditions entre l'ADN de HPV-IP6 et l'ADN du HPV32 (31) a permis d'aligner les cartes physiques de ces deux génomes et de définir la position théorique des différents gènes portés par l'ADN de l'HPV42 (IP6), comme indiqué ci-après et par rapport a l'origine de la carte de la fig. 11 et en pourcentages de longueur.

| Localisation putative des principaux gènes et de la région intergénique (NC) du HPV42 (IP6) | |
|---|---|
| | HPV42 (IP6) |
| E6-E7 | 11,5-21,5 |
| E1 | 21,5-46,5 |
| E2 | 45-59,5 |
| L2 | 63-82,5 |
| L1 | 80,5-15 |
| NC | 1,5-11,5 |

L'utilisation de sondes radioactives préparées à partir de l'ADN HPV-IP6 purifié a permis de déterminer le pouvoir pathogène de ce virus.

Des prélèvements de lésions prolifératives bénignes (condylomes, papillomes) ou de néoplasies intraépithéliales des organes génitaux externes, de la région périanale et du col utérin provenant de 163 malades ont été analysés. L'ADN du HPV42 (IP6) a été détecté chez 10 malades présentant des lésions bénignes (condylomes, papillomes).

Au cours d'une étude de 1522 prélèvements de cellules cervico-vaginales, destinée à préciser la fréquence de l'infection de l'épithélium du col utérin par un HPV, l'ADN du HPV42 (IP6) a été mis en évidence dans 9 prélèvements correspondant à une cytologie normale ou inflammatoire ou à une dysplasie légère sur 110 prélèvements contenant un ADN d'HPV.

Le HPV42 (IP6) constitue donc un type additionnel d'HPV génital relativement fréquent, ne possédant vraisemblablement qu'un potentiel oncogène faible. Il est donc souhaitable de l'incorporer à tous mélanges d'ADNs de HPV destinés à la préparation de sondes moléculaires, en vue du diagnostic ou du dépistage des types d'HPV sexuellement transmis, agents de tumeurs génitales bénignes, ne constituant pas un risque majeur pour le développement de néoplasies génitales et, en particulier, de cancers du col de l'utérus.

Etant donné la grande diversité des HPVs susceptibles d'entre isolés à partir des différentes formes de verrues ou autres lésions cutanées ou de muqueuses, il est cependant préféré d'utiliser, pour le diagnostic de chaque type d'affection mentionné dans le tableau, des mélanges comportant plus d'un ou deux ADN-HPVs, dès lors que d'autres ADN-HPVs ont été reconnus comme pouvant également intervenir dans le développement du même type d'affection. Le diagnostic de la nature de l'infection et de son évolution possible sera d'autant plus efficace que le nombre de sondes utilisées sera plus élevé. En outre, des essais d'hybridation réalisés avec ds mélanges différents de sondes permettra des diagnostics différentiels présentant un degré de probabilité également plus important de la nature du mal dont souffre le patient.

L'invention concerne donc plus particulièrement encore des mélanges ou cocktails ou compositions contenant l'ADN-HPV39 (IP5) et/ou l'ADN-HPV42 (IP6) seuls ou en mélange avec d'autres ADNs-HPVs (ou sondes contenant ces ADNs-HPVs ou de séquences de ces derniers), susceptibles d'être utilisés en combinaison pour réaliser des diagnostics globaux des différentes formes d'infections à papillomavirus, éventuellement aux fins de pronostiquer l'évolution possible de l'infection.

Des mélanges préférés conformes à l'invention sont identifiés dans le tableau ci-après :

TABLEAU

CARACTERISTIQUES DES MELANGES D'ADN D'HPV UTILISABLES POUR LE DIAGNOSTIC VIROLOGIQUE D'INFECTIONS A PAPILLOMAVIRUS

| Désignation des mélanges | Constitution (type d'HPV) 1) | Maladies à diagnostiquer |
|---|---|---|
| 1 | 1,2d ,4 | Verrues cutanées ou muqueuses (en particulier, verrues vulgaires et plantaires). Diagnostic différentiel de l'épidermodysplasie verruciforme. |
| 2 | 3,10a,10b ,28 ,29 | Verrues planes ou intermédiaires cutanées ou muqueuses. Néoplasies intraépithéliales et cancers cutanés. Diagnostic différentiel de l'épidermodysplasie verruciforme. |
| 3 | 5,17a , 24 | Epidermodysplasie verruciforme. Néoplasies intra-épithéliales et cancers cutanés. |
| 4 | 5,8,12,14a ,14b ,19 ,20 ,21 ,22 ,23, 36 (IP4) | Epidermodysplasie verruciforme. |
| 5 | 9,15 ,17a ,17b | Epidermodysplasie verruciforme. |
| 6 | 24 | Epidermodysplasie verruciforme. |
| 7 | 5,8,14b ,34(32), 36 (IP4) | Cancers cutanés de l'épidermodysplasie verruciforme. Néoplasies intraépithéliales et cancers cutanés. |
| 8 | 13, 32(31) | Hyperplasie épithéliale focale orale ; Diagnostic différentiel des néoplasies intraépithéliales orales. |
| 9 | 34(32), 36 (IP4) | Néoplasies intraépithéliales et cancers cutanés. |
| 10 | 16, 18, 33 (IP2), 39 (IP5) | Néoplasies génitales et cancers du col de l'utérus |
| 11 | 6, 11, 42 (IP6) | Condylomes, papillomes |

Le tableau susdit indique également les natures des affections susceptibles d'être plus particulièrement diagnostiquées par l'utilisation des mélanges figurant dans la partie gauche du tableau. Il est rappelé que les cartes de restriction des autres ADN-HPVs identifiés dans le tableau qui précède sont contenues dans les figures 1 à 9.

Il est à remarquer que HPV39 (IP5) peut être considérés comme particulièrement représentatif de sondes utilisables pour la détection des risques de développpement de cancers du col de l'utérus et le IP6 comme représentatif des sondes utilisables pour la détection des infections à papillomes bénignes.

L'ADN de HPV42 (IP6) peut être associé à l'ADN de HPV-6 et/ou HPV11.

Enfin l'invention concerne aussi le mélange de toutes les sondes.

De même l'invention concerne plus particulièrement un cocktail de sondes contenant l'ADN des HPV spécifiques des cancers génitaux, ce cocktail contenant :

HPV33 (IP2)

HPV39 (IP5)

HPV42 (IP6)

HPV6

HPV11

HPV16

HPV18

HPV32 (31)

HPV35.

HPV42 (IP6) est représentatif de sondes utilisables pour la détection d'infections à papillomavirus bénignes. L'ADN de l'HPV42 (IP6) peut être associé à l'ADN de HP6 et/ou de HPV11.

L'invention concerne donc plus particulièrement encore des trousses ou "kits" de diagnostic comprenant au moins 11 groupes figurant dans les groupes numérotés de 1 à 11 dans le tableau sous la rubrique "Désignation des mélanges".

Dans ce qui précède, on a surtout envisagé l'utilisation, à titre de sondes, des ADN-HPVs entiers clonés. Ceux-ci peuvent cependant être substitués par des fragments clonés de ces différents ADNs, notamment par les gènes E1 ou L1 et par les gènes E6-E7.

Le principe de base des détections in vitro d'ADN-HPV mettra naturellement en jeu des hybridations opérées dans des conditions strictes ou moins strictes. On peut opérer par exemple comme suit, étant naturellement entendu que les essais de diagnostic décrits ne sauraient être considérés comme limitatifs des conditions d'emploi des sondes ou mélanges de sondes selon l'invention.

L'objet des examens mettant en jeu des sondes préparées à partir de mélanges d'ADNs de HPVs clonés est de mettre en évidence un HPV et d'identifier le type d'HPV dans une biopsie, dans des cellules obtenues par grattage de lésions, ou dans des coupes de biopsies fixées par exemple par le mélange de Carnoy (éthanol, chloroforme, acide acétique 6:3:1) et incluses dans la paraffine. L'examen nécessite l'extraction préalable de l'ADN des prélèvements selon des méthodes dont le principe est connu et met en jeu l'analyse de cet ADN par des expériences d'hybridation moléculaire, effectuées dans des conditions strictes ou moins strictes, à l'aide de sondes radioactives (marquées au $^{32}$P ou au $^{35}$S) préparées à partir de mélanges d'ADN-HPVs. Chaque examen requiert, en général, l'utilisation de plusieurs mélanges de sondes.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou Genescreenplus), l'hybridation de chaque membrane, dans les conditions usuelles, avec un mélange de sondes et la détection des hybrides radioactifs par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, Genescreenplus) et leur hybridation, dans les conditions usuelles, avec différents mélanges de sondes. La formation d'hybrides radioactifs est détectée après exposition des membranes au contact d'un film radiographique.

On peut aussi avoir recours à une méthode d'hybridation in situ, par exemple sur ces coupes de tissus fixés par le mélange de Carnoy et incluses dans la paraffine.

Les sondes radioactives sont constituées soit par des ADNs d'HPVs marqués par la méthode de "nick-translation", soit par des ARNs préparés par transcription d'ADNs viraux insérés dans un vecteur, par exemple de type pSP6. L'utilisation de sondes radioactives présente l'avantage d'une grande sensibilité, mais ceci n'exclut pas l'utilisation de sondes non radioactives par exemple biotinylées et susceptibles d être reconnues par des anticorps soit marqués eux-mêmes, soit eux-mêmes reconnus par des anticorps portant un marqueur enzymatique, fluorescent, etc..

L'invention concerne donc encore des nécessaires ou "kits" contenant une pluralité des sondes sus-indiquées, et comprennent :

- HPV39 (IP5) et HPV42 (IP6) respectivement pris de façons isolées,
- les sondes précédentes, en mélange avec d'autres, notamment celles définies plus haut,

ces "kits" étant destinées à des études de diagnostic in vitro par hybridation entre des préparations virales obtenues à partir de patients et les divers groupes ou mélanges.

Comme il va de soi et comme il résulte d'ailleurs déjà de ce qui précède, l'invention ne se limite nullement à ceux de ses modes d'application et de réalisation qui ont été plus spécialement envisagés ;

elle en embrasse au contraire toutes les variantes ; notamment la référence dans les revendications à une désignation ADN-HPV suivie d'un nombre déterminé, et à laquelle correspond un ADN-HPV dont la carte de restriction a été fournie dans les dessins, s'entend comme signifiant que ces revendications couvrent tous les ADN-HPVs qui ont en commun avec cet ADN-HPV particulier de pouvoir être classés dans le même type, selon la définition qui en a été donnée plus haut, et a fortiori aux ADN-HPV appartenant au même sous-type.

On notera que les ADNs recombinants désignés ci-après ont été déposés le 21 janvier 1986 à la C.N.C.M. (Collection Nationale des Cultures de Micro-Organismes de l'INSTITUT PASTEUR de Paris), sous les numéros apparaissant ci-après :

pSP65/HPV39 (IP5) (plasmide dans E. coli) : n° I-507
pSP64/HPV42 (IP6) (plasmide dans E. coli) : n° I-508

BIBLIOGRAPHIE

(1) Dürst, M. et al., 1983, Proc. Natl. Acad. Sci. U.S.A., 80:3812-3815.
(1bis) Bospart M. et al., 1984, Embo J. 3 = 1151-1157
(2) Coggin, J.R., Jr. et al., 1979, Cancer Res., 39:545-546.
(3) Gissmann, L. et al., 1982. J. Virol. 44:393-400.
(4) Green, M. et al., 1982, Proc. Natl. Acad. Sci. U.S.A. 79:4437-4441.
(5) Heilman, C.A. et al., 1980, Virol. 36:395-407.
(6) Jablonska, S. et al., 1972, Cancer Res., 32:583-589.
(7) Jablonska. S. et al., 1982, Springer Semin. Immunopathol. 5:33-62.
(8) Kremsdorf, D. et al., 1982, J. Virol. 43:436-447.
(9) Kremsdorf, D. et al., 1983, J. Virol. 48:340-351.
(10) Lutzner, M.A. et al., 1978, Bull. Cancer, 65:169-182.
(11) Lutzner, M.A. et al., 1983, Lancet ii:422-424.
(12) Migozzi, M. et al., 1965 Bull. Soc. Franc. Derm. Syph. 72:747-748.
(13) Orth, G. et al., 1980, Cold Spring Harbor Conf. Cell Proliferation, 7:259-282.
(14) Orth, G. et al., 1981, J. Invest. Dermatol. 76:97-102.
(15) Orth, G. et al., 1979, Cancer Res. 39:1074-1082.
(16) Ostrow, R.S. et al, 1982, Proc. Natl. Acad. Sci. U.S.A., 79:1634-1638.
(17) Ostrow, R.S. et al., 1983, Ann. Acad. Dermatol. 8:398-404.
(18) Pfister, H. et al., 1983, Cancer Res. 43:1436-1441.
(19) Pfister, H. et al., 1983, J. Virol. 47:363-366.
(20) Pfister, H. et al., 1981. Int. J. Cancer, 27:645-650.
(21) Rueda, L.A. et al., 1976, Med. Cut. I.L.A. 2:113-136.
(22) Ruiter, M. et al., J. Invest. Dermatol., 47:247-252.
(23) Sutcliffe, J.G., 1978, Nucleic Acids Res. 5:2721-2728.
(24) Tsumori, T. et al., 1983, J. Gen. Virol. 64:967-969.

Les éléments bibliographiques qui suivent doivent encore être pris en considération (certaines références sont précédées des désignations de certains HPV, lorsque ceux-ci ont été décrits pour la première fois par les auteurs correspondants) :

HPV18 :     BOSHART M. et al., 1984, EMBO J., 3:1151-1157,
HPV-IP2 :     BEAUDENON S. et al., 1986, Nature,321:246,249,
           BEAUDENON S. et al., 1987, J. Inv. Dermatol., 88 N° 1,
           KAWASHIMA M. et al., 1986a, J. Virol., 57:688-692,
           KAWASHIMA M. et al., 1986b, Virology, 154:389-394,
HPV6 :     DE VILLIERS E.M. et al., 1981, J.Virol., 40:932-935,
HPV11 :     GISSMANN L. et al., 1982, J. Virol., 44:393-400,
HPV31 :     LORINCZ A. et al., 1986a, J. Virol., 58:225-229,
HPV35 :     LORINCZ A. et al., 1986b, Bambury report, 21:225-237,
HPV16 :     DURST et al., 1983.

**Revendications**

**Revendications pour les Etats contractants suivants : BE, CH, DE, FR, GB, GR, IT, LI, LU, NL, SE**

**1.** ADN-HPV ayant une taille comprise entre environ 7000 et environ 8000 paires de bases ou un fragment de cet ADN-HPV, cet ADN-HPV étant obtenu à partir d'un des papillomavirus répondant aux désignations HPV39 (IP5) et HPV42 (IP6).

2.  Fragment d'ADN selon la revendication 1, caractérisé en ce qu'il correspond aux gènes E1, E6-E7, L1 ou L2 ou qu'il correspond encore à un fragment correspondant à une région intergénique dudit ADN-HPV.

3.  ADN recombinant contenant un ADN-HPV selon la revendication 1 ou un fragment selon la revendication 2.

4.  ADN, fragment ou ADN recombinant selon l'une quelconque des revendications 1, 2 ou 3 pour l'utilisation en tant que sonde de détection d'ADN-HPVs.

5.  Composition contenant en mélange des ADN-HPVs, utilisable pour la détection de papillomavirus dans un milieu biologique le contenant, caractérisée en ce qu'elle contient un ADN-HPV conforme à la revendication 1, associé à un ou plusieurs ADN-HPVs distincts.

6.  Composition selon la revendication 5, caractérisée en ce qu'elle contient en mélange les ADN-HPVs
    33 (IP2)
    39 (IP5)
    42 (IP6)
    6
    11
    16
    18
    32 (31)
    35.

7.  Composition selon la revendication 5, caractérisée en ce qu'elle contient l'ADN-HPV42 (IP6) en mélange avec l'ADN-HPV6 ou avec l'ADN-HPV11 ou avec les deux à la fois.

8.  Procédé de préparation d'une sonde de détection d'un ADN-HPV, caractérisé par le clonage d'un vecteur dans un hôte particulier, tel qu'un micro-organisme procaryote, ce vecteur ayant préalablement été modifié, par recombinaison in vitro, par l'ADN-HPV de la revendication 1 ou le fragment d'ADN de la revendication 2 et d'un vecteur approprié, pour obtenir un vecteur recombinant contenant l'ADN ou le fragment d'ADN dérivé du virus dans un site de ce vecteur permettant son clonage ultérieur dans cet hôte, et par la récupération et la purification et, le cas échéant, le marquage de l'ADN recombinant cloné obtenu.

9.  Nécessaire ou kit comportant neuf groupes de sondes ou mélanges de sondes distinctes, ces groupes (1 à 9) étant respectivement constitués
    1) d'au moins l'ADN-HPV 2d
    2) d'au moins l'un des ADN-HPVs 20b, 28 et 29,
    3) d'au moins l'un des ADN-HPVs 17, 24,
    4) d'au moins l'un des ADN-HPVs 14, 15, 17, 19, 20, 21, 22, 23 et 36 (IP4),
    5) d'au moins l'un des ADN-HPVs 15 et 17,
    6) de l'ADN de HPV24,
    7) d'au moins l'un des ADN-HPVs 14, 34 (32) et 36 (IP4),
    8) de l'ADN-HPV 32 (31),
    9) de l'ADN-HPV 34 (32), caractérisé en ce qu'il comporte en outre deux autres groupes (10 et 11) constituès
    10) l'ADN-HPV39 (IP5) seul ou en mélange avec au moins l'un des ADN-HPVs 16, 18, et 33 (IP2)
    11) l'ADN-HPV42 (IP6) seul ou en mélange avec au moins l'un des ADN-HPVs 6 et 11,
    étant entendu que les ADNs des 11 groupes sont choisis de façon à être en toutes circonstances différents les uns des autres dans la mesure où chacun des 11 groupes serait réduit à un seul des ADNs qui le composent.

10. Nécessaire ou kit pour le diagnostic différencié in vitro entre différents types de verrues et hyperplasies répondant à des pronostics variables, s'étageant entre la conservation du caractère bénin de ces verrues et des formes susceptibles de dégénérer en néoplasies intra-épithéliales et en cancers de la peau et des muqueuses, ce kit comportant neuf groupes de sondes recombinantes contenant des insérats d'ADNs de papillomavirus, ces groupes (1 à 9) étant respectivement préparés à partir:

1) d'au moins l'un des ADN-HPVs 1, 2d et 4

2) d'au moins l'un des ADN-HPVs 3, 10a, 10b, 28, et 29

3) d'au moins l'un des ADN-HPVs 5, 17a et 24

4) d'au moins l'un des ADN-HPVs 5, 8, 12, 14a, 14b, 19, 20, 21, 22, 23 et 36(IP4)

5) d'au moins l'un des ADN-HPVs 9, 15, 17a et 17b

6) de l'ADN-HPV 24

7) d'au moins l'un des ADN-HPVs 5, 8, 14b, 34(32), et 36(IP4)

8) d'au moins l'un des ADN-HPVs 13 et 32(31)

9) d'au moins l'un des ADN-HPVs 34(32) et 36(IP4)

caractérisé en ce qu'il comporte un outre deux autres groupes (10 et 11) préparés à partir:

10) de l'ADN-HPV 39(IP5) seul ou en mélange avec au moins l'un des ADN-HPVs 16, 18 et 33(IP2),

11) de l'ADN-HPV 42(IP6) seul ou en mélange avec au moins l'un des ADN-HPVs 6 et 11,

,étant entendu que les ADNs des 11 groupes sont choisis de façon à être en toutes circonstances différents les uns des autres dans la mesure où chacun des 11 groupes serait réduit à un seul des ADNs qui le composent, ces sondes permettant alors, par les hybridations auxquelles certains de ces mélanges sont susceptibles de donner lieu avec la préparation d'ADN provenant du patient concerné, d'assurer la détection de l'existence ou de la potentialité de l'une des maladies suivantes :

1) Verrues cutanées ou muqueuses (en particulier, verrues vulgaires et plantaires). Diagnostic différentiel de l'épidermodysplasie verruciforme.

2) Verrues planes ou intermédiaires cutanées ou muqueuses. Néoplasies intraépithéliales et cancers cutanés. Diagnostic différentiel de l'épidermodysplasie verruciforme.

3) Epidermodysplasie verruciforme. Néoplasies intra-épithéliales et cancers cutanés.

4) Epidermodysplasie verruciforme.

5) Epidermodysplasie verruciforme.

6) Epidermodysplasie verruciforme.

7) Cancers cutanés de l'épidermodysplasie verruciforme. Néoplasies intra-épithéliales et cancers cutanés.

8) Hyperplasie épithéliale focale orale ; diagnostic différentiel des néoplasies intra-épithéliales orales.

9) Néoplasies intra-épithéliales et cancers cutanés.

10) Néoplasies génitales et cancers du col de l'utérus.

11) Condylomes, papillomes.

**11.** Procédé de détection et d'identification de papillomavirus contenus dans un échantillon biologique comprenant la réalisation d'essais d'hybridation avec un ADN-HPV, fragment d'ADN ou ADN recombinant selon la revendication 3 ou avec une composition selon l'une quelconque des revendications 5 à 7, et la détection de ceux des ADN-HPVs préalablement obtenus à partir desdits échantillons biologiques, qui donnent lieu à hybridation.

**12.** Procédé selon la revendication 11, appliqué à la détection in vitro de risques de cancers du col de l'utérus, caractérisé en ce que l'ADN-HPV, fragment d'ADN ou ADN recombinant mis en oeuvre est issu de HPV39 (IP5).

**13.** Procédé selon la revendication 11, appliqué à la détection in vitro de risques de cancers génitaux, caractérisé en ce que la composition mise en oeuvre est celle de la revendication 6.

**14.** Procédé selon la revendication 11, appliqué à la détection in vitro d'infections à papillomavirus bénignes, caractérisé en ce que l'ADN-HPV, fragment d'ADN ou ADN recombinant mis en oeuvre est issu de HPV42 (IP6).

**15.** Procédé selon la revendication 11, appliqué à la détection in vitro d'infections à papillomavirus bénignes, caractérisé en ce que la composition mise en oeuvre est celle de la revendication 7.

**Revendications pour les Etats contractants suivants : AT, ES**

1. Procédé de préparation d'un ADN-HPV ayant une taille comprise entre environ 7000 et environ 8000 paires de bases ou un fragment de cet ADN-HPV, comprenant le clonage d'un vecteur dans un hôte approprié, tel qu'un micro-organisme procaryote, ce vecteur ayant préalalablement été modifié par recombinaison in vitro, par tout ou partie d'un ADN d'un virus choisi parmi les papillomavirus répondant aux désignations HPV39 (IP5) et HPV42 (IP6), pour obtenir un vecteur recombinant contenant cet ADN ou fragment d'ADN dans un site de ce vecteur permettant son clonage ultérieur dans l'hôte susdit, et par la récupération et la purification et, le cas échéant, le marquage de l'ADN recombinant cloné obtenu.

2. Procédé de fabrication d'un fragment d'ADN selon la revendication 1, caractérisé en ce que ce vecteur a préalablement été modifié par recombinaison in vitro par un fragment choisi parmi les gènes E1, E6-E7, L1 ou L2 ou un fragment correspondant à une région intergénique dudit ADN-HPV.

3. ADN, fragment ou ADN recombinant fabriqué par un procédé selon la revendication 1 ou 2, pour l'utilisation en tant que sonde de détection d'ADN-HPVs.

4. Composition contenant en mélange des ADN-HPVs, utilisable pour la détection de papillomavirus dans un milieu biologique le contenant, caractérisée en ce qu'elle contient un ADN-HPV fabriqué par le procédé conforme à la revendication 1, associé à un ou plusieurs ADN-HPVs distincts.

5. Composition selon la revendication 4, qui contient en mélange les ADN-HPVs :
   33 (IP2)
   6
   11
   16
   18
   32 (31)
   35,
   caractérisée en ce qu'elle contient aussi les ADN-HPVs 39 (IP5) et 42 (IP6) fabriqués par le procédé conforme à la revendication 1.

6. Composition selon la revendication 5, caractérisée en ce qu'elle contient l'ADN-HPV42 (IP6) en mélange avec l'ADN-HPV6 ou avec l'ADN-HPV11 ou avec les deux à la fois.

7. Nécessaire ou kit comportant neuf groupes de sondes ou mélanges de sondes distinctes, ces groupes 1 à 9 étant respectivement constitués :
   1) d'au moins l'ADN-HPV2d,
   2) d'au moins l'un des ADN-HPVs 20b, 28 et 29,
   3) d'au moins l'un des ADN-HPVs 17 et 24,
   4) d'au moins l'un des ADN-HPVs 14, 15, 17, 19, 20, 21, 22, 23 et 36 (IP4),
   5) d'au moins l'un des ADN-HPVs 15 et 17,
   6) de l'ADN-HPV24,
   7) d'au moins l'un des ADNs-HPVs 14, 34 (32) et 36 (IP4),
   8) de l'ADN-HPV32 (31),
   9) de l'ADN-HPV34 (32),
   caractérisé en ce qu'il comporte en outre deux autres groupes (10 et 11) constitués,
   10) de l'ADN-HPV39 (IP5) fabriqué par le procédé selon la revendication 1, seul ou en mélange avec au moins l'un des ADN-HPVs 16, 18, et 33 (IP2)
   11) de l'ADN-HPV42 (IP6) fabriqué par le procédé selon la revendication 1, seul ou en mélange avec au moins l'un des ADN-HPVs 6 et 11,
   étant entendu que les ADNs des 11 groupes sont choisis de façon à être en toutes circonstances différents les uns des autres dans la mesure où chacun des 11 groupes serait réduit à un seul des ADNs qui le composent.

8. Nécessaire ou kit pour le diagnostic différencié in vitro entre différents types de verrues et hyperplasies répondant à des pronostics variables, s'étageant entre la conservation du caractère bénin de ces verrues et des formes susceptibles de dégénérer en néoplasies intra-épithéliales et en cancers de la peau et des muqueuses, ce kit comportant neuf groupes de sondes recombinantes contenant des insérats d'ADN de papillomavirus; ces groupes 1 à 9 étant préparés à partir :

1) d'au moins l'un des ADN-HPVs 1, 2d, 4

2) d'au moins l'un des ADN-HPVs 3, 10a, 10b, 28, 29

3) d'au moins l'un des ADN-HPVs 5, 17a, 24

4) d'au moins l'un des ADN-HPVs 5, 8, 12, 14a, 14b, 19, 20, 21, 22, 23, 36 (IP4)

5) d'au moins l'un des ADN-HPVs 9, 15, 17a, 17b

6) de l'ADN-HPVs 24

7) d'au moins l'un des ADN-HPVs 5, 8,14b, 32, 36 (IP4)

8) d'au moins l'un des ADN-HPVs 13, 32 (31)

9) d'au moins l'un des ADN-HPVs 34 (32), 36 (IP4)

caractérisé en ce qu'il comporte en outre deux autres groupes (10 et 11)

10) de l'ADN-HPV39 (IP5) fabriqué selon le procédé de la revendication 1, seul ou en mélange avec au moins l'un des ADN-HPVs 16, 18, 33 (IP2)

11) de l'ADN-HPV 42 (IP6), fabriqué selon le procédé de la revendication 1, seul ou en mélange avec au moins l'un des ADN-HPVs 6 et 11,

étant entendu que les ADNs des 11 groupes sont choisis de façon à être en toutes circonstances différents les uns des autres dans la mesure où chacun des 11 groupes serait réduit à un seul des ADNs qui le composent, ces sondes permettant alors, par les hybridations auxquelles certains de ces mélanges sont susceptibles de donner lieu avec la préparation d'ADN provenant du patient concerné, d'assurer la détection de l'existence ou de la potentialité de l'une des maladies suivantes :

1) Verrues cutanées ou muqueuses (en particulier, verrues vulgaires et plantaires). Diagnostic différentiel de l'épidermodysplasie verruciforme.

2) Verrues planes ou intermédiaires cutanées ou muqueuses. Néoplasies intraépithéliales et cancers cutanés. Diagnostic différentiel de l'épidermodysplasie verruciforme.

3) Epidermodysplasie verruciforme. Néoplasies intra-épithéliales et cancers cutanés.

4) Epidermodysplasie verruciforme.

5) Epidermodysplasie verruciforme.

6) Epidermodysplasie verruciforme.

7) Cancers cutanés de l'épidermodysplasie verruciforme. Néoplasies intra-épithéliales et cancers cutanés.

8) Hyperplasie épithéliale focale orale ; diagnostic différentiel des néoplasies intra-épithéliales orales.

9) Néoplasies intra-épithéliales et cancers cutanés.

10) Néoplasies génitales et cancers du col de l'utérus.

11) Condylomes, papillomes.

**9.** Procédé de détection et d'identification de papillomavirus contenus dans un échantillon biologique comprenant la réalisation d'essais d'hybridation avec un ADN-HPV, fragment d'ADN ou ADN recombinant fabriqué selon le procédé de la revendication 1 ou 2, ou avec une composition selon l'une quelconque des revendications 4 à 6, et la détection de ceux des ADN-HPVs préalablement obtenus à partir desdits échantillons biologiques, qui donnent lieu à hybridation.

**10.** Procédé selon la revendication 9, appliqué à la détection in vitro de risques de cancers du col de l'utérus, caractérisé en ce que l'ADN-HPV, fragment d'ADN ou ADN recombinant mis en oeuvre est issu de HPV39 (IP5).

**11.** Procédé selon la revendication 9, appliqué à la détection in vitro de risques de cancers génitaux, caractérisé en ce que la composition mise en oeuvre est celle de la revendication 5.

**12.** Procédé selon la revendication 9, appliqué à la détection in vitro d'infections à papillomavirus bénignes, caractérisé en ce que l'ADN-HPV, fragment d'ADN ou ADN recombinant mis en oeuvre est issu de HPV42 (IP6) et obtenu par le procédé selon la revendication 1 ou 2.

**13.** Procédé selon la revendication 11, appliqué à la détection in vitro d'infections à papillomavirus bénignes, caractérisé en ce que la composition mise en oeuvre est celle de la revendication 6.

**Claims**
**Claims for the following Contracting States : BE, CH, DE, FR, GB, GR, IT, LI, LU, NL, SE**

1. DNA-HPV having a size included between about 7000 and about 8000 base pairs or a fragment of this DNA-HPV, this DNA-HPV being obtained from a papillomavirus corresponding to the designations HPV39 (IP5) and HPV42 (IP6).

2. DNA fragment according to Claim 1, characterized in that it corresponds to the genes E1, E6-E7, L1 or L2 or in that it corresponds to a fragment corresponding to an intergenic region of the said DNA-HPV.

3. Recombinant DNA containing a DNA-HPV according to Claim 1 or a fragment according to Claim 2.

4. DNA fragment or recombinant DNA according to any one of the Claims 1, 2 or 3 for use as a probe for the detection of DNA-HPVs.

5. Composition containing a mixture of DNA-HPVs which can be used for the detection of a papillomavirus in a biological medium containing it, characterized in that it contains a DNA-HPV in conformity with Claim 1, in association with one or more distinct DNA-HPVs.

6. Composition according to Claim 5, characterized in that it contains a mixture of the DNA-HPVs 33 (IP2); 39 (IP5); 42 (IP6); 6; 11; 16; 18; 32 (31); 35.

7. Composition according to Claim 5, characterized in that it contains the DNA-HPV42 (IP6) as a mixture with the DNA-HPV6 or with the DNA-HPV11 or with both together.

8. Procedure for the preparation of a detection probe for a DNA-HPV, characterized by the cloning of a vector in a specific host, such as a prokaryotic microorganism, this vector having been modified beforehand by in vitro recombination by the DNA-HPV of Claim 1 or the DNA fragment of Claim 2 and a suitable vector in order to obtain a recombinant vector containing the DNA or the DNA fragment derived from the virus at a site on this vector making possible its subsequent cloning in this host, and by the recovery and purification and, where necessary, the labelling of the cloned recombinant DNA produced.

9. Kit containing nine groups of probes or mixtures of distinct probes, these groups (1 to 9) being constituted, respectively, by
   1) at least the DNA-HPV 2d
   2) at least one of the DNA-HPVs 20b, 28 and 29,
   3) at least one of the DNA-HPVs 17, 24,
   4) at least one of the DNA-HPVs 14, 15, 17, 19, 20, 21, 22, 23 and 36 (IP4),
   5) at least one of the DNA-HPVs 15 and 17,
   6) the DNA-HPV24,
   7) at least one of the DNA-HPVs 34 (32) and 36 (IP4),
   8) the DNA-HPV32 (31)
   9) the DNA-HPV34 (32),
   characterized in that it contains in addition two other groups (10 and 11) constituted by
   10) the DNA-HPV39 (IP5) alone or as a mixture with at least one of the DNA-HPVs 16, 18 and 33 (IP2)
   11) the DNA-HPV 42 (IP6) alone or as a mixture with at least one of the DNA-HPVs 6 and 11,
   it being understood that the DNAs of the 11 groups are selected so that under all circumstances they are different from each other even if each of the 11 groups were to be reduced to only one of the DNAs of which it is composed.

10. Kit for the in vitro diagnosis to differentiate between different types of warts and hyperplasias corresponding to variable prognoses, which range from the preservation of the benign character of these warts to forms capable of degenerating into intra-epithelial neoplasias and cancers of the skin and mucous membranes, this kit containing nine groups of recombinant probes containing DNA inserts of papillomaviruses, these groups (1 to 9) being prepared respectively from:
   1) at least one of the DNA-HPVs 1, 2d and 4

14

2) at least one of the DNA-HPVs 3, 10a, 10b, 28 and 29

3) at least one of the DNA-HPVs 5, 17a and 24

4) at least one of the DNA-HPVs 5, 8, 12, 14a, 14b, 19, 20, 21, 22, 23 and 36 (IP4)

5) at least one of the DNA-HPVs 9, 15, 17a and 17b

6) the DNA-HPV 24

7) at least one of the DNA-HPVs 5, 8, 14b, 34(32), and 36 (IP4)

8) at least one of the DNA-HPVs 13 and 32 (31)

9) at least one of the DNA-HPVs 34(32) and 36 (IP4)

characterized in that it contains in addition to other groups (10 and

11) prepared from:

10) the DNA-HPV 39 (IP5) alone or as a mixture with at least one of the DNA-HPVs 16, 18 and 33-(IP2),

11) the DNA-HPV 42(IP6) alone or as a mixture with at least one of the DNA-HPVs 6 and 11,

it being understood that the DNAs of the 11 groups are selected so that under all circumstances they are different from each other even if each of the 11 groups were to be reduced to only one of the DNAs of which it is composed, these probes then making it possible to ensure the detection of the existence or potentiality of one of the following diseases as a result of the hybridizations to which some of these mixtures are capable of giving rise with the DNA preparation derived from the patient concerned:

1) Cutaneous warts or warts of the mucous membranes (in particular, verrucae and plantar warts). Differential diagnosis of epidermodysplasia verruciformis.

2) Plane warts or intermediary cutaneous or mucosal warts. Intra-epithelial neoplasias and cutaneous cancers. Differential diagnosis of epidermodysplasia verruciformis.

3) Epidermodysplasia verruciformis. Intra-epithelial neoplasias and cutaneous cancers.

4) Epidermodysplasia verruciformis.

5) Epidermodysplasia verruciformis.

6) Epidermodysplasia verruciformis.

7) Cutaneous cancers of the epidermodysplasia verruciformis. Intra-epithelial neoplasias and cutaneous cancers.

8) Oral focal epithelial hyperplasia; differential diagnosis of oral intra-epithelial neoplasias.

9) Intra-epithelial neoplasias and cutaneous cancers.

10) Genital neoplasias and cancers of the uterine cervix.

11) Condylomas, papillomas.

**11.** Procedure for the detection and identification of papillomaviruses contained in a biological sample, comprising the carrying out of hybridization assays with a DNA-HPV, a DNA fragment or a recombinant DNA according to Claim 3 or with a composition according to any one of the Claims 5 to 7, and the detection of those DNA-HPVs obtained from the said biological samples which give rise to hybridization.

**12.** Procedure according to Claim 11, applied to the in vitro detection of risks of cancer of the uterine cervix, characterized in that the DNA-HPV, DNA fragment or recombinant DNA used is derived from HPV39 (IP5).

**13.** Procedure according to Claim 11, applied to the in vitro detection of risks of genital cancers, characterized in that the composition used is that of Claim 6.

**14.** Procedure according to Claim 11, applied to the in vitro detection of benign papillomavirus infections, characterized in that the DNA-HPV, DNA fragment or recombinant DNA used is derived from HPV42 (IP6).

**15.** Procedure according to Claim 11, applied to the in vitro detection of benign papillomavirus infections, characterized in that the composition used is that of Claim 7.

**Claims for the following Contracting States : AT, ES**

**1.** Procedure for the preparation of a DNA-HPV with a size included between about 7000 and about 8000 base pairs or a fragment of this DNA-HPV, comprising the cloning of a vector in a suitable host, such as a prokaryotic microorganism, this vector having been modified beforehand by means of in vitro recombination by all or part of a DNA of a virus selected from the papillomaviruses answering to the designations HPV39 (IP5) and HPV42 (IP6), to obtain a recombinant vector containing this DNA or DNA fragment at a site on this vector making possible its subsequent cloning in the above-mentioned host, and by the recovery and purification and, where appropriate, the labelling of this cloned recombinant DNA obtained.

2. Procedure for the preparation of a DNA fragment according to Claim 1, characterized in that this vector has been modified beforehand by means of in vitro recombination by a fragment selected from the genes E1, E6-E7, 11 or 12 or a fragment corresponding to an intergenic region of the said DNA-HPV.

3. DNA fragment or recombinant DNA prepared by a procedure according to Claim 1 or 2 for use as a detection probe of DNA-HPVs.

4. Composition containing a mixture of DNA-HPVs which can be used for the detection of a papillomavirus in a biological sample containing it, characterized in that it contains a DNA-HPV prepared by the procedure according to Claim 1, associated with one or several distinct DNA-HPVs.

5. Composition according to Claim 4, which contains the following mixture of DNA-HPVs:
33 (IP2)
6
11
16
18
32 (31)
35,
characterized in that it also contains the DNA-HPVs 39 (IP5) and 42 (IP6) prepared by the procedure according to Claim 1.

6. Composition according to Claim 5, characterized in that it contains the DNA-HPV42 (IP6) in a mixture with the DNA-HPV6 or with the DNA-HPV11 or with both together.

7. Kit containing 9 groups of probes or mixtures of distinct probes, these groups 1 to 9 being constituted respectively by:
1) the DNA-HPV2d,
2) at least one of the DNA-HPVs 20b, 28 and 29,
3) at least one of the DNA-HPVs 17 and 24,
4) at least one of the DNA-HPVs 14, 15, 17, 19, 20, 21, 22, 23 and 36 (IP4),
5) at least one of the DNA-HPVs 15 and 17,
6) the DNA-HPV24,
7) at least one of the DNA-HPVs 14, 34 (32) and 36 (IP4),
8) the DNA-HPV32 (31),
9) the DNA-HPV34 (32),
characterized in that it contains in addition two other groups (10 and 11) constituted by:
10) the DNA-HPV39 (IP5) prepared by the procedure according to Claim 1, alone or in a mixture with at least one of the DNA-HPVs 16, 18 and 33 (IP2)
11) the DNA-HPV42 (IP6) prepared by the procedure according to Claim 1, alone or in a mixture with at least one of the DNA-HPVs 6 and 11,
it being understood that the DNAs of the 11 groups are selected so that under all circumstances they are different from each other even if each of the 11 groups were to be reduced to only one of the DNAs of which it is composed.

8. Kit for the in vitro diagnosis to differentiate between different types of warts and hyperplasias corresponding to variable prognoses, ranging from the preservation of the benign character of these warts to forms capable of degenerating into intra-epithelial neoplasias and cancers of the skin and mucous membranes, this kit containing nine groups of recombinant probes containing DNA inserts of papillomaviruses; these groups (1 to 9) being prepared from :
1) at least one of the DNA-HPVs 1, 2d, 4
2) at least one of the DNA-HPVs 3, 10a, 10b, 28, 29

3) at least one of the DNA-HPVs 5, 17a, 24

4) at least one of the DNA-HPVs 5, 8, 12, 14a, 14b, 19, 20, 21, 22, 23, 36 (IP4)

5) at least one of the DNA-HPVs 9, 15, 17a, 17b

6) the DNA-HPV 24

7) at least one of the DNA-HPVs 5, 8, 14b, 32, 36 (IP4)

8) at least one of the DNA-HPVs 13, 32 (31)

9) at least one of the DNA-HPVs 34 (32), 36 (IP4)

characterized in that it contains in addition two other groups (10 and 11)

10) the DNA-HPV39 (IP5) prepared according to the procedure of Claim 1, alone or in a mixture with at least one of the DNA-HPVs 16, 18, 33 (IP2)

11) the DNA-HPV42 (IP6) prepared according to the procedure of Claim 1, alone or as a mixture with at least one of the DNA-HPVs 6 and 11,

it being understood that the DNAs of the 11 groups are selected so that under all circumstances they are different from each other even if each of the 11 groups were to be reduced to only one of the DNAs of which it is composed, these probes then making it possible to ensure the detection of the existence or potentiality of one of the following diseases as a consequence of the hybridizations to which some of these mixtures are capable of giving rise with the DNA preparation derived from the patient concerned:

1) Cutaneous warts or warts of the mucous membranes (in particular, verrucae and plantar warts). Differential diagnosis of epidermodysplasia verruciformis.

2) Plane warts or intermediary cutaneous or mucosal warts. Intra-epithelial neoplasias cutaneous cancers. Differential diagnosis of epidermodysplasia verruciformis.

3) Epidermodysplasia verruciformis. Intra-epithelial neoplasias and cutaneous cancers.

4) Epidermodysplasia verruciformis.

5) Epidermodysplasia verruciformis.

6) Epidermodysplasia verruciformis.

7) Cutaneous cancers of epidermodysplasia verruciformis. Intra-epithelial neoplasias and cutaneous cancers.

8) Oral focal epithelial hyperplasia; differential diagnosis of oral intra-epithelial neoplasias.

9) Intra-epithelial neoplasias and cutaneous cancers.

10) Genital neoplasias and cancers of the uterine cervix.

11) Condylomas, papillomas.

9. Procedure for the detection and identification of papillomaviruses contained in a biological sample comprising the carrying out of hybridization assays with a DNA-HPV, a DNA fragment or a recombinant DNA prepared according to the procedure of Claim 1 or 2, or with a composition according to any one of the Claim 4 to 6, and the detection of those DNA-HPVs previously obtained from the said biological samples which give rise to hybridization.

10. Procedure according to Claim 9, applied to the in vitro detection of risks of cancers of the uterine cervix, characterized in that the DNA-HPV, DNA fragment or recombinant DNA used is derived from HPV39 (IP5).

11. Procedure according to Claim 9, applied to the in vitro detection of risks of genital cancers, characterized in that the composition used is that of Claim 5.

12. Procedure according to Claim 9, applied to the in vitro detection of benign papillomavirus infections, characterized in that the DNA-HPV, DNA fragment or recombinant DNA used is derived from HPV42 (IP6) and obtained by the procedure according to Claim 1 or 2.

13. Procedure according to Claim 11, applied to the in vitro detection of benign papillomavirus infections, characterized in that the composition used is that of Claim 6.

**Patentansprüche**

**Patentansprüche für folgende Vertragsstaaten : BE, CH, DE, FR, GB, GR, IT, LI, LU, NL, SE**

1. HPV-DNA mit einer Länge von ca. 7000 bis ca. 8000 Basenpaaren oder ein Fragment dieser HPV-DNA, wobei diese HPV-DNA aus einem der Papillomaviren mit den Bezeichnungen HPV39 (IP5) und HPV42 (IP6) erhalten ist.

**2.** DNA-Fragment nach Anspruch 1, dadurch **gekennzeichnet,** daß es den Genen E1, E6-E7, L1 oder L2 entspricht oder daß es auch einem Fragment entsprechend der intergenetischen Region der HPV-DNA entspricht.

**3.** Rekombinante DNA, enthaltend eine HPV-DNA nach Anspruch 1 oder ein Fragment nach Anspruch 2.

**4.** DNA, Fragment oder rekombinante DNA nach einem der Ansprüche 1, 2 oder 3, zur Verwendung als Sonde für den Nachweis von HPV-DNAs.

**5.** Zusammensetzung, enthaltend eine Mischung von HPV-DNAs, zur Verwendung für den Nachweis von Papillomavirus in einem biologischen Milieu, das es enthält, dadurch **gekennzeichnet,** daß sie eine HPV-DNA nach Anspruch 1 zusammen mit einer oder mehreren verschiedenen HPV-DNAs enthält.

**6.** Zusammensetzung nach Anspruch 5, dadurch **gekennzeichnet,** daß sie die Mischung der HPV-DNAs enthält

33 (IP2)

39 (IP5)

42 (IP6)

6

11

16

18

32 (31)

35.

**7.** Zusammensetzung nach Anspruch 5, dadurch **gekennzeichnet,** daß sie HPV42(IP6)-DNA in einer Mischung mit HPV6-DNA oder mit HPV11-DNA oder mit die beiden zugleich enthält.

**8.** Verfahren zur Herstellung einer Sonde zum Nachweis einer HPV-DNA, **gekennzeichnet** durch Klonierung eines Vektors in einem bestimmten Wirt, wie einem procaryontischen Mikroorganismus, wobei der Vektor zuvor durch in vitro Rekombination durch die HPV-DNA nach Anspruch 1 oder dem DNA-Fragment nach Anspruch 2 und einem geeigneten Vektor unter Erhalt eines rekombinanten Vektors modifiziert wurde, der die DNA oder das DNA-Fragment aus dem Virus in einer Schnittstelle des Vektors enthält, was seine spätere Klonierung im Wirt erlaubt, und durch Wiedergewinnung und Reinigung und, gegebenenfalls Markierung des erhaltenen rekombinanten DNA-Klons.

**9.** Ausrüstung oder Kit, das neue Gruppen von Sonden oder Mischungen verschiedener Sonden enthält, wobei die Gruppen (1 bis 9) entsprechend bestehen aus

1) mindestens einer DNA von HPV2d,

2) mindestens einer der DNAs von HPV20b, 28 und 29,

3) mindestens einer der DNAs von HPV17 und 24,

4) mindestens einer der DNAs von HPV14, 15, 17, 19, 20, 21, 22, 23 und 36 (IP4),

5) mindestens einer der DNAs von HPV15 und 17,

6) der DNA von HPV24,

7) mindestens eine der DNA von HPV14, 34 (32) und 36 (IP4),

8) der DNA von HPV32 (31),

9) der DNA von HPV 34 (32),

dadurch **gekennzeichnet,** daß es außerdem zwei andere Gruppen (10 und 11) enthält, bestehend aus:

10) der DNA von HPV39 (IP5) allein oder in Mischung mit mindestens einer der DNAs von HPV16, 18 und 33 (IP2),

11) der DNA von HPV42 (IP6) allein oder in Mischung mit mindestens einer der DNAs von HPV6 und 11, mit der Maßgabe, daß die DNAs aus den 11 Gruppen derart ausgewählt sind, daß in jedem Fall die einen von den anderen in dem Maße verschieden sind wie jede der 11 Gruppen auf nur eine der DNAs, die sie enthalten, beschränkt ist.

**10.** Ausrüstung oder Kit zur Diagnose, die in vitro zwischen verschiedenen Typen von Warzen und Hyperplasien unterscheidet, die sich durch veränderliche Anzeichen äußern, die sich abstufen vom Erhalt eines gutartigen Charakters dieser Warzen bis zu Formen, die zur Entwicklung intra-epithelialer Neoplasien und Hautkrebs und Schleimhautkrebs in der Lage sind, wobei das Kit neun Gruppen von rekombinanten Sonden enthält, die Einschübe von DNAs von Papillomavirus enthalten, wobei die Gruppen entsprechend hergestellt sind aus

1) mindestens einer der DNAs von HPV1, 2d, 4,

2) mindestens einer der DNAs von HPV3, 10a, 10b, 28 und 29,

3) mindestens einer der DNAs von HPV5, 17a und 24,

4) mindestens einer der DNAs von HPV5, 8, 12, 14a, 14b, 19, 20, 21, 22, 23 und 36 (IP4),

5) mindestens einer der DNAs von HPV9, 15, 17a und 17b,

6) der DNA von HPV24,

7) mindestens einer der DNAs von HPV5, 8, 14b, 34 (32) und 36 (IP4),

8) mindestens einer der DNAs von HPV13 und 32 (31),

9) mindestens einer der DNAs von HPV34 (32) und 36 (IP4),

dadurch **gekennzeichnet,** daß es außerdem zwei andere Gruppen (10 und 11) enthält, bestehend aus:

10) der DNA von HPV39 (IP5) allein oder in Mischung mit mindestens einer der DNAs von HPV16, 18 und 33 (IP2),

11) der DNA von HPV42 (IP6) allein oder in Mischung mit mindestens einer DNA von HPV6 und 11, mit der Maßgabe, daß die DNAs aus den 11 Gruppen derart ausgewählt sind, daß in jedem Fall die einen von den anderen in dem Maße

verschieden sind wie jede der 11 Gruppen auf nur eine der DNAs, die sie enthalten, beschränkt ist, wobei die Sonden in diesem Fall durch Hybridisierung von bestimmten Mischungen, die hierzu in der Lage sind, mit der DNA Präparation aus dem betreffenden Patienten, den sicheren Nachweis erlauben, daß eine der folgenden Krankheiten vorliegt oder vorliegen könnte:

1) Haut- oder Schleimhautwarzen (insbesondere gewöhnliche oder Sohlenwarzen). Diagnose unterscheidet warzenartige Epidermodysplasie

2) Flache oder intermediäre Haut- oder Schleimhautwarzen, intra-epitheliale Neoplasien und Hautkrebs. Diagnose unterscheidet warzenartige Epidermodysplasie

3) Warzenartige Epidermodysplasie, intra-epitheliale Neoplasien und Hautkrebs

4) Warzenartige Epidermodysplasie

5) Warzenartige Epidermodysplasie

6) Warzenartige Epidermodysplasie

7) Hautkrebs von warzenartiger Epidermodysplasie, intra-epitheliale Neoplasien und Hautkrebs

8) Fokale orale epitheliale Hyperplasie; Diagnose unterscheidet von intra-epithelialen oralen Neoplasien

9) intra-epitheliale Neoplasien und Hautkrebs

10) Genitale Neoplasien und Gebärmutterhalskrebs

11) Condylome, Papillome.

**11.** Verfahren zum Nachweis und Identifizierung von Papillomavirus, enthalten in einer biologischen Probe, umfassend die Durchführung von Hybridisierungsversuchen mit einer HPV-DNA, einem DNA-Fragment oder einer rekombinanten DNA nach Anspruch 3 oder mit einer Zusammensetzung nach einem der Ansprüche 5 bis 7 und Nachweis der von den zuvor aus diesen biologischen Flüssigkeiten erhaltenen HPV-DNAs, die eine Hybridisierung ergeben.

**12.** Verfahren nach Anspruch 11, angewandt auf den in vitro Nachweis des Gebärmutterhalskrebsrisikos, dadurch **gekennzeichnet,** daß die HPV-DNA, ein Fragment von der DNA, oder rekombinante DNA hervorgegangen aus HPV39 (IP5), eingesetzt wird.

**13.** Verfahren nach Anspruch 11, angewandt auf den in vitro Nachweis des genitalen Krebsrisikos, dadurch **gekennzeichnet,** daß die eingesetzte Zusammensetzung diejenige aus Anspruch 6 ist.

**14.** Verfahren nach Anspruch 11, angewandt auf den in vitro Nachweis von gutartigen Papillomavirusinfektionen, dadurch **gekennzeichnet,** daß die eingesetzte HPV-DNA, das Fragment der DNA oder die rekombinante DNA von HPV42 (IP6) abstammt.

**15.** Verfahren nach Anspruch 11, angewandt auf den in vitro Nachweis von gutartigen Papillomavirusinfektionen, dadurch **gekennzeichnet,** daß die eingesetzte Zusammensetzung diejenige aus Anspruch 7 ist.

**Patentansprüche für folgende Vertragsstaaten : AT, ES**

1. Verfahren zur Herstellung einer HPV-DNA mit einer Länge von ca. 7000 bis ca. 8000 Basenpaaren oder ein Fragment dieser HPV-DNA, umfassend die Klonierung eines Vektors in einem geeigneten Wirts, wie z. B. einem procaryontischen Mikroorganismus, wobei der Vektor zuvor durch in vitro Rekombination durch die gesamte oder einen Teil der Virus-DNA, ausgewählt aus Papillomaviren mit den Bezeichnungen HPV39 (IP5) und HPV42 (IP6) modifiziert wurde, unter Erhalt eines rekombinanten Vektors, der diese DNA oder das DNA-Fragment in einer Stelle des Vektors enthält, was seine spätere Klonierung in dem Wirt erlaubt, und die Wiedergewinnung und Reinigung und gegebenenfalls, Markierung des erhaltenen rekombinanten DNA-Klons.

2. Verfahren zur Herstellung eines DNA-Fragments nach Anspruch 1, dadurch **gekennzeichnet,** daß der Vektor zuvor durch in vitro Rekombination mit einem Fragment, ausgewählt aus den Genen E1, E6-E7, L1 oder L2 oder einem Fragment entsprechend einer intergenetischen Region der HPV-DNA, modifiziert wurde.

3. DNA-Fragment oder rekombinante DNA hergestellt durch ein Verfahren nach Anspruch 1 oder 2 zur Verwendung als Sonde zum Nachweis von HPV-DNAs.

4. Zusammensetzung enthaltend eine Mischung von HPV-DNAs zur Verwendung für den Nachweis von Papillomavirus in einem biologischen Milieu, das es enthält, dadurch **gekennzeichnet,** daß sie eine HPV-DNA hergestellt durch das Verfahren gemäß Anspruch 1 zusammen mit einer oder mehreren verschiedenen HPV-DNAs enthält.

5. Zusammensetzung nach Anspruch 4, die die Mischung der HPV-DNAs enthält:
   33 (IP2)
   6
   11
   16
   18
   32 (31)
   35
   dadurch **gekennzeichnet,** daß sie auch die DNAs von HPV39 (IP5) und 42 (IP6), hergestellt durch das Verfahren nach Anspruch 1, enthält.

6. Zusammensetzung nach Anspruch 5, dadurch **gekennzeichnet,** daß sie HPV42(IP6)-DNA zusammen mit HPV6-DNA oder mit HPV11-DNA oder mit den beiden zusammen enthält.

7. Ausrüstung oder Kit, das neue Gruppen von Sonden oder Mischungen verschiedener Sonden enthält, wobei die Gruppen (1 bis 9) wie folgt zusammengesetzt sind aus:
   1) mindestens einer DNA von HPV2d,
   2) mindestens einer der DNAs von HPV20b, 28 und 29,
   3) mindestens einer der DNAs von HPV17 und 24,
   4) mindestens einer der DNAs von HPV14, 15, 17, 19, 20, 21, 22, 23 und 36 (IP4),
   5) mindestens einer der DNAs von HPV15 und 17,
   6) der DNA von HPV24,
   7) mindestens einer der DNAs von HPV14, 34 (32) und 36 (IP4),
   8) der DNA von HPV32 (31),
   9) der DNA von HPV 34 (32),
   dadurch **gekennzeichnet,** daß sie außerdem zwei andere Gruppen (10 und 11) enthält, bestehend aus:
   10) HPV39 (IP5)-DNA, hergestellt durch das Verfahren nach Anspruch 1, allein oder in Mischung mit mindestens einer der DNAs der HPV16, 18 und 33 (IP2), und
   11) HPV42 (IP6)-DNA, hergestellt durch das Verfahren nach Anspruch 1, allein oder in Mischung mit mindestens einer der DNAs der HPV6 und 11,
   mit der Maßgabe, daß die DNAs aus den 11 Gruppen derart ausgewählt sind, daß in jedem Fall die einen von den anderen in dem Maße verschieden sind wie jede der 11 Gruppen auf nur eine der DNAs, die sie enthalten, beschränkt ist.

**8.** Ausrüstung oder Kit zur Diagnose, die in vitro zwischen verschiedenen Typen von Warzen und Hyperplasien unterscheidet, die sich durch veränderliche Anzeichen äußern, die sich abstufen vom Erhalt eines gutartigen Charakters dieser Warzen bis zu Formen, die zur Entwicklung intra-epithelialer Neoplasien und Hautkrebs und Schleimhautkrebs in der Lage sind, wobei das Kit neun Gruppen von rekombinanten Sonden enthält, die Einschübe von DNAs von Papillomavirus enthalten, wobei die Gruppen entsprechend hergestellt sind aus

1) mindestens einer der DNAs von HPV1,2d, 4,

2) mindestens einer der DNAs von HPV3, 10a, 10b, 28 und 29,

3) mindestens einer der DNAs von HPV5, 17a und 24,

4) mindestens einer der DNAs von HPV5, 8, 12, 14a, 14b, 19, 20, 21, 22, 23 und 36 (IP4),

5) mindestens einer der DNAs von HPV9, 15, 17a und 17b,

6) der DNA von HPV24,

7) mindestens einer der DNAs von HPV5, 8, 14b, 34 (32) und 36 (IP4),

8) mindestens einer der DNAs von HPV13 und 32 (31),

9) mindestens einer der DNAs von HPV34 (32) und 36 (IP4),

dadurch **gekennzeichnet,** daß es außerdem zwei andere Gruppen (10 und 11) enthält, bestehend aus:

10) der HPV39 (IP5)-DNA, hergestellt durch das Verfahren nach Anspruch 1, allein oder in Mischung mit mindestens einen DNAs der HPV16, 18 und 33 (IP2), und

11) der HPV42 (IP6)-DNA, hergestellt durch das Verfahren nach Anspruch 1, allein oder in Mischung mit mindestens einer DNA der HPV6 und 11, mit der Maßgabe, daß die DNAs aus den 11 Gruppen derart ausgewählt sind, daß in jedem

Fall die einen von den anderen in dem Maße verschieden sind wie jede der 11 Gruppen auf nur eine der DNAs, die sie enthalten, beschränkt ist, wobei die Sonden in diesem Fall durch Hybridisierung von bestimmten Mischungen, die hierzu in der Lage sind, mit der DNA Präparation aus dem betreffenden Patienten, den sicheren Nachweis erlauben, daß eine der folgenden Krankheiten vorliegt oder vorliegen könnte:

1) Haut- oder Schleimhautwarzen (insbesondere gewöhnliche oder Sohlenwarzen). Diagnose unterscheidet warzenartige Epidermodysplasie

2) Flache oder intermediäre Haut- oder Schleimhautwarzen, intra-epitheliale Neoplasien und Hautkrebs. Diagnose unterscheidet warzenartige Epidermodysplasie

3) Warzenartige Epidermodysplasie, intra-epitheliale Neoplasien und Hautkrebs

4) Warzenartige Epidermodysplasie

5) Warzenartige Epidermodysplasie

6) Warzenartige Epidermodysplasie

7) Hautkrebs von warzenartiger Epidermodysplasie, intra-epitheliale Neoplasien und Hautkrebs

8) Fokale orale epitheliale Hyperplasie; Diagnose unterscheidet von intra-epithelialen oralen Neoplasien

9) intra-epitheliale Neoplasien und Hautkrebs

10) Genitale Neoplasien und Gebärmutterhalskrebs

11) Condylome, Papillome.

**9.** Verfahren zum Nachweis und Identifizierung von Papillomavirus, enthalten in einer biologischen Probe, umfassend die Durchführung von Hybridisierungsversuchen mit einer HPV-DNA, einem Fragment der DNA oder einer rekombinanten DNA hergestellt nach dem Verfahren gemäß den Ansprüchen 1 oder 2 oder mit einer Mischung nach einem der Ansprüche 4 bis 6, und Nachweis dieser zuvor aus den biologischen Flüssigkeiten erhaltenen HPV-DNAs, die eine Hybridisierung ergeben.

**10.** Verfahren nach Anspruch 9 angewandt auf den in vitro Nachweis des Gebärmutterhalskrebsrisikos, dadurch **gekennzeichnet,** daß die HPV-DNA, ein Fragment von der DNA oder rekombinante DNA, hervorgegangen aus HPV39 (IP5), eingesetzt wird.

**11.** Verfahren nach Anspruch 9 angewandt auf den in vitro Nachweis des genitalen Krebsrisikos, dadurch **gekennzeichnet,** daß die eingesetzte Zusammensetzung diejenige nach Anspruch 5 ist.

**12.** Verfahren nach Anspruch 9 angewandt auf den in vitro Nachweis von gutartigen Papillomavirusinfektionen, dadurch **gekennzeichnet,** daß die eingesetzte HPV-DNA, das Fragment der DNA oder die rekombinante DNA von HPV42 (IP6) abstammt und erhalten wird das Verfahren nach den Ansprüchen 1 oder 2.

**13.** Verfahren nach Anspruch 11 angewandt auf den in vitro Nachweis von gutartigen Papillomavirusinfektionen, dadurch **gekennzeichnet,** daß die eingesetzte Zusammensetzung diejenige aus Anspruch 6 ist.

FIG.1

**HPV 1a**

- Bam HI
- Hind III
- Pst I
- Pst I
- Pvu II
- Sac I
- Hind III
- Bgl II
- Eco RI
- Pvu II
- Bgl II
- Hind II-Hpa I
- Eco RI
- Hind II-Hpa I
- Bgl II
- Bgl II
- Bgl I
- Hind III
- Hind II

**HPV 2d**

- Eco RI
- Hind II
- Hind II
- Bgl I
- Pvu II
- Pst I
- Hind II-Hpa I
- Hind II
- Bam HI
- Pvu II
- Pst I
- Bgl I
- Bam HI
- Pst I
- Pvu II
- Pvu II
- Pst I

**HPV 4a**

- 0
- Bam HI
- Hind II
- Hind III
- Hind II
- 10
- Eco RI
- Hind II
- 20
- 30
- Hind III
- 40
- Hind II
- 50
- 60
- Eco RI
- Bgl I
- 70
- Hind II-Hpa I
- 80
- Hind III
- 90
- 100

FIG.2

FIG.3

FIG.4a

HPV 14b   HPV 14a   HPV 12   HPV 8   HPV 5a

**HPV 14b**
★Bam HI
Hind III
EcoRI-Pvu
★Pst I
Hind
★Hind III
★Hpal-Hind II
EcoRI
Pst I
EcoRI
★Pvu II
★Bgl II
Pst I
Hpal-Hind II
Bgl II
Bgl II
Pst I
Bgl II
Pst I
★Ava I
★Hind II
★Bgl I
★Pst I
Pst I
★Hind II
★Hpal-Hind II
★Ava I

**HPV 14a**
Bam HI ★
Pst I ★
Hind III ★
Hind III
Hind II-Hpa I ★
Hind II-Hpa I
Bam HI
Pst I
Eco RI
Pvu II ★
Bgl II ★
Bgl II
Bgl II
Ava I ★
Hind II ★
Bgl I ★
Pst I ★
Hind II ★
Hind II-Hpal ★
Sac I
Ava I ★

**HPV 12**
Hind III
Pvu II
Eco RI
Pvu II
Pst I
Hind II
Pst I
Pvu II
Hind II
Hind II-Sal I
Hind II-Hpal
Bgl I
Sac I
Bgl II
Pvu II
Pvu II
Pst I
Pst I
Sac I
Hind II-Sal I
Bgl I
Pvu I
Ava I-Smal
Pst I
Hind II-Hpal
Eco RI

**HPV 8**
Bam HI
Bgl II
Pvu II
Ava I-Smal
Hind II-Hpa I
Pst I
Bgl I
Pvu II
Pst I
Hind II-Hpa I
Hind II
Hind II
Hind II-Hpa
Pst I
Eco RI
Bgl I
Bgl II
Pst I
Hind II
Pvu II

**HPV 5a**
Sac I
Hind II
Eco RI
Hind III
Hind II-Hpa I
Hind II-Hpal
Hind II
Bgl I
Hind II-Hpa I
Pvu II
Bgl II
Bam HI
Ava I
Hind II
Hind II-Hpal
Hind II
Pst I
Ava I
Ava I
Hind II-Sal I
Pst I
Pst I
Eco RI
Pvu II
Ava I
Bam HI
Bgl II
Pst I
Hind III

0
10
20
30
40
50
60
70
80
90
100

FIG.4b

HPV.23

Bam HI
Eco RI
Hind II
Ava I
Hind II-Hpa I
Bgl I
Hind II
Bgl II
Pst I
Sac I
Hind III
Eco RI
Hind II
Pst I
Sac I

Bgl I

Hind II
Pvu II

Eco RI

Pst I
Bgl II
Ava I

Bgl II
Pst I

HPV.22

Sac I

Bgl II
Pst I

Hind II
Eco RI

Hind II
Bgl II
Pst I
Hind II-Hpa I
Hind III
Ava I
Hind II

Ava I

Pst I
Bgl II
Hind III

Ava I
Pst I

Pst I
Ava I-Sma I
Hind II

Hind III
Hind II

Hind III

HPV.21

Bam HI
Pvu II
Hind III
Hind II
Hind III

Hind II-Hpa I
Hind II
Hind II-Hpa I
Pst I
Bgl I

Pst I

Bgl II

Pst I
Eco RI
Pst I
Bgl II
Pst I

Hind II
Hind II
Hind II-Sal I
Bgl I
Pvu I

Hind II-Hpa I

Pst I
Pvu I
Eco RI
Pst I
Hind II

Ava I

HPV.20

Ava I
Pvu II
Hind III
Pst I
Bam HI
Hind II
Hind III

Hind II-Hpa I

Pst I
Hind II-Hpa I
Pvu II
Bgl I
Pst I
Pst I

Bgl II
Bgl II
Pst I
Hind II

Bgl II

Hind II

Pvu II
Bgl I
Bam HI

Hind II

Hind II
Hind II-Hpa I

Bam HI
Hind II-Hpa I

HPV.19

Bam HI
Pst I

Pvu I

Hind II
Pvu II
Pst I
Pst I

Pvu II
Ava I
Hind II

Hind III
Bgl II
Hind II
Hind III

Pvu II
Hind II-Hpa I
Pvu II
Bgl II
Hind II

Eco RI

Bgl II

EP 0 235 004 B1

FIG.5

EP 0 235 004 B1

FIG.6

HPV-24 ●

BamH I

Hind III
Pvu II
Ava I
Hind II
Bgl I
Pst I
Hind III
Hind II
Bgl II
Hind II–Hpa I
Hind III–Hpa I

Pst I

Pst I
Bgl II
Pst I
Hind II
Hind II
Pst I
Hind III
Hind III
Pst I
BamH I
Ava I
Pvu II
Bgl II

Hind II

| 0 | 10 | 20 | 30 | 40 | 50 | 60 | 70 | 80 | 90 | 100 |

FIG.7

FIG.8

HPV33
(HPV_IP2)  FIG.9  HPV36
(HPV_IP4)

Bgl II (0)
Hind III (3.8)
Pst I (8.6)
Kpn I (12.5)

Pvu II (22.5)
Pst I (23.8)
Pst I (25.7)
Hind II (26.7)
Hind III (29.8)
Xba I (30.2)

Kpn I (40.4)

Bgl I (47.6)
Pst I (51.4)

Pvu II (73.5)
Hind II (74.3)
Ava I - Sma I (76.9)

Hind II - Npa I (85.7)
Hind II - Npa I (89.5)

X ba I (96.2)
Bgl II (100)

0
10
20
30
40
50
60
70
80
90
100

Eco RI
Pst I
Bgl I

Pst I
Kpn I
Pst I

Kpn I

Pvu I

Hinc II
Pst I
Hinc II - Hpa I

Kpn I
Bam HI
Pst I
Ava I
Hinc II
Sac I
Pst I
Hind III
Pvu II
Hinc II

Bam HI

HPV39

(IP 5)

```
EcoRI (0)
PstI (1.0)
Hind II (2.9)

PstI (14.4)

Hind II  Sal I (20.2)
PstI (22.8)
Pvu II (23.3)

Pvu II (30.1)
Bam HI (30.8)
Hind III (34.3)
PstI (38.2)

KpnI (46.2)

PstI (50.1)

KpnI (57.7)

KpnI (60.6/66.4)

KpnI (69.3)

BamHI (74.1)
Ava I (75.0)
Kpn I (76.0)

Xba I (86.5)

PstI (94.2)

EcoRI (100)
```

# FIG.10

HPV42

(IP 6)

```
BamHI (0)
XbaI (4.7)
PstI (5.7)
AccI (6.9)
Hind III (7.3)
Hind II (8.2)
Eco RI (10.5)

Acc I (19.9)
Bgl II (21.2)

Eco RV (37.1)
PstI (39.2)

BglI (46.2)

PstI (53.0)

Pst I (66.8)
Hind II (68.0)
Ssp I (69.9)
Eco RV (71.4)
Xba I (74.5)
PstI (76.2)
SspI (77.7)
AccI (79.8)
PstI (80.0)

KpnI (91.8)
Acc I (93.1)
Pvu II (96.1)
Ssp I (96.1)
Bam HI (100)
```

# FIG.11